# EUROPEAN PATENT APPLICATION

(11) **EP 4 424 822 A1**
(43) Date of publication of application: **04.09.2024**
(21) Application number: 23159044.9
(22) Date of filing: 28.02.2023
(51) Int. Cl.: C12N 9/12, A61K 35/17, A61K 48/00, A61P 31/12, A61P 35/02, C12N 5/0789, C12N 15/09

(54) **DESIGNER RECOMBINASE FOR THE PRECISE EXCISION OF HTLV-1-PROVIRUS**

(71) Applicant: PROVIREX Genome Editing Therapies GmbH, 22547 Hamburg (DE); Technische Universität Dresden, 01062 Dresden (DE)
(72) Inventor: Buchholz, Frank, 01307 Dresden (DE); Rojo Romanos, Teresa, 01097 Dresden (DE); Beschorner, Niklas, 20255 Hamburg (DE)
(74) Representative: Moré, Solveig Helga

(57) **Abstract**

The present invention relates to a nucleic acid encoding a recombinase capable of recombining asymmetric target sequences of SEQ ID NO:1 within the long terminal repeat of proviral DNA of a plurality of HTLV-1 strains, the recombinase having specific amino acid exchanges compared to the sequence of cre recombinase. The invention also provides expression vectors comprising said nucleic acids and cells comprising the same, the recombinase in protein form and pharmaceutical compositions comprising them. A method for provision of such recombinases is also provided. The invention also provides a method of treating a HTLV-1-infected subject, wherein the subject optionally has adult T-cell leukemia (ATL) and/or HTLV-1-associated myelopathy (HAM).

## Description

The present invention relates to a nucleic acid encoding a recombinase capable of recombining asymmetric target sequences of SEQ ID NO: 1 within the long terminal repeat of proviral DNA of a plurality of HTLV-1 strains, the recombinase having specific amino acid exchanges compared to the sequence of cre recombinase. The invention also provides expression vectors comprising said nucleic acids and cells comprising the same, the recombinase in protein form and pharmaceutical compositions comprising them. A method for provision of such recombinases is also provided. The invention also provides a method of treating a HTLV-1 - infected subject, wherein the subject optionally has adult T-cell leukemia (ATL) and/or HTLV-1-associated myelopathy (HAM).

Retroviral infections remain a challenge for modem medicine, mainly because there are still no effective therapies to cure them. The permanent integration of the proviral DNA into the host cell genome results in lifelong infection. For this reason, from the over 37 million Human Immunodeficiency Virus (HIV)-infected people worldwide, the ones with access to therapy are dependent on lifelong drug treatment that suppress the virus but cannot cure the patients from the infection. A promising approach to solve this problem is the use of programmable DNA nucleases to compromise the virus. While some nuclease strategies aim to target the viral entry receptors of host cells, others focus on directly disrupting the genetic material of the virus. The use of designer-nucleases to treat HIV-1 infections has been possible because the integrated proviral DNA serves as a targetable substrate for genome scissors such as zinc finger nucleases (ZNFs), transcription activator-like effector nucleases (TALENs) or CRISPR/Cas9. However, nucleases induce double strand breaks and rely on the cellular DNA repair machinery, which processes them in an unpredictable manner. Indeed, the use of nucleases to target viral sequences leads to the rapid formation of resistant clones, potentially compromising the utility of this approach (Wang *et al.,* 2016a, 2016b, Yoder & Bundschuh 2016, De Silva Feelixge *et al,* 2016). Another class of genome editing tools, namely site-specific recombinases (SSRs), circumvent this problem, as they do not rely on host DNA repair mechanisms and edit DNA in a seamless manner (Meinke *et al,* 2016). However, adapting SSRs to recombine custom chosen sequences poses challenges. Nevertheless, directed evolution methods have proven useful to redirect the specificity of recombinases to recognize new target sequences (Buchholz & Stewart, 2001; Santoro & Schultz, 2002; Eroshenko & Church, 2013). In fact, designer-recombinases have been developed as powerful tools for the removal of the HIV-1 provirus, with high efficiency and precision (Hauber *et al,* 2013; Karpinski *et al,* 2016; Sarkar *et al,* 2007, WO 2008/08391, WO 2011/147590, WO 2016/034553). Importantly, these designer-recombinases have demonstrated their anti-retroviral activity without the emergence of resistant clones in humanized mouse models, indicating that they have considerable therapeutic potential (Hauber *et al,* 2013; Karpinski *et al,* 2016).

Targeting infections using genome editing tools is not limited to the HIV virus and similar approaches could potentially be utilized for treating other human retroviral infections. Human T-cell leukaemia virus type 1 (HTLV-1) is a human-pathogenic retrovirus that causes an aggressive neoplasia, adult T-cell leukaemia (ATL), in about 5% of carriers (*Martin et al.,* 2018). Furthermore, in a smaller percentage of infected individuals, an inflammatory neurological disease called HTLV-1-associated myelopathy/tropical spastic paraparesis (HAM/TSP) is observed. Approximately 10 million people are infected with HTLV-1 around the world with a high percentage of them in endemic areas that include, among others, Japan, Central Australia, South America, the Caribbean and Sub-Saharan Africa. The lack of effective therapies for ATL and HAM/TSP denote the need for new treatment approaches. Viral transmission occurs mostly through cell-to-cell spread at tight cell contacts or via cellular protrusions. Because of this mechanism of infection and viral amplification by clonal expansion, HTLV-1 has a remarkably stable genome, and there is little sequence variation between individuals and even within cells of an infected person (Furukawa *et al,* 2001; Wattel *et al,* 1995). The HTLV-1 proviral genome is approximately 9kb and it is flanked by 5' and 3' long terminal repeats that are identical in sequence (Boxus & Willems, 2009). The genetic sequence stability of the virus and the fact that the provirus integrates in the genome of infected cells make HTLV-1 a suitable candidate for treatment with gene editing tools. Indeed, different groups have already attempted the disruption of the virus using nucleases: Tanaka *et al.* used zinc finger nucleases to disrupt the LTR promoter and Nakagawa *et al.* used CRISPR/Cas9 to target the Hbz gene, which led to a reduction in cell proliferation in 3 different ATL cell lines (Tanaka *et al,* 2013; Nakagawa *et al,* 2018). However, with the use of nucleases it is likely that, as for HIV-1, resistant HTLV-1 clones will emerge.

In light of this, the present inventors addressed the problem of providing an advantageous enzyme capable of excising the HTLV-1 provirus from infected cells, which, e.g., overcomes at least one of these disadvantages. This problem is solved by the claimed subject-matter.

In particular, the invention provides a nucleic acid encoding a tailored recombinase capable of recombining asymmetric target sequences of SEQ ID NO:1 within the long terminal repeat of proviral DNA of a plurality of HTLV-1 strains, wherein the amino acid sequence of the tailored recombinase has at least 70% sequence identity to a sequence according to SEQ ID NO:13, wherein said tailored recombinase comprises, compared to SEQ ID NO:11, at least the amino acid exchanges selected from the group consisting ofP15L, S38P, A84T, Q90K, S108G, K122R, I166V, A175S, N245Y, E266G and T268A.

The inventors were able to identify a conserved loxP-like sequence (loxHTLV, SEQ ID NO: 1) present in the long terminal repeats of the majority of virus isolates, which can therefore advantageously be used for excision of the provirus. After 181 cycles of substrate-linked molecular evolution (SLiDE), the inventors isolated designer-recombinases able to recognize loxHTLV, and to efficiently recombine the loxHTLV sequence in bacteria and human cells with high specificity. Expression of these recombinases in human T-cells resulted in antiviral activity when challenged with an HTLV-1 infection. Moreover, expression of these recombinases in chronically infected SP cells led to the excision of HTLV-1 proviral DNA. Their data show that recombinase mediated excision of the HTLV-1 provirus represents a promising strategy to reverse HTLV-1 infections that can be therapeutically used.

As the prototype recombinase on which the recombinase of the invention is originally based is cre, the sequence of the claimed recombinase is provided in comparison to the sequence of cre in SEQ ID NO: 11. This applies both when the substituted amino acid is defined (e.g., P15L means that the P in position 15 of SEQ ID NO: L is substituted by L), or when only the position and the amino acid present in said position are specified (e.g., 15L also means that the P in position 15 of SEQ ID NO: L is substituted by L. As defined herein, further amino acids can be substituted compared to the cre sequence, but they can also be the same as in cre.

SEQ ID NO: 13 is the amino acid sequence of a particularly preferred, high activity recombinase of the invention that is also designated RecHTLV or G9 (which is used synonymously).

P15L, S38P, A84T, Q90K, S108G, K122R, I166V, A175S, N245Y, E266G and T268A are present in all recombinases of the invention and not present in cre. S38P, A84T, Q90K, E266G are substitutions that have not previously been identified in the art and that thus seem to be important for recognition of the HTLV-1 target sequence. Other substitutions also appear in the prior art recombinases Brec-1 or Tre that have been found to target asymmetric target sequences in HIV-1. All of these amino acid substitutions are considered essential substitutions for the recombinases of the invention. The invention provides nucleic acids, e.g., expression vectors, encoding all recombinases of the invention.

The term "recombinase" as used herein refers to a protein involved in recombination. As such recombinases recognize and bind two specific DNA sequences termed "recombination sites" or "target sites" and mediate recombination between these two target sites. Accordingly, the term "recombinase" is meant to refer to any protein component of any recombinant system that mediates DNA rearrangements in a specific DNA locus. Naturally occurring recombinases recognize symmetric target sites consisting of two identical sequences termed "half- site" of approximately 9-20 bp forming an inverted repeat, wherein the half-site sequences are separated by a spacer sequence of 5-12 bp. Recombinases from the tyrosine integrase family are characterized by having a tyrosine as the active site nucleophile that is utilized for DNA cleavage, whereas recombinases from the serine integrase family use a serine instead of a tyrosine. The recombinase of the present invention has been modified or tailored to be able to recombine asymmetric target sequence. It is therefore also designated a designer recombinase or a tailored recombinase.

Preferably, the nucleic acid of the invention encodes a tailored recombinase having at least 80%, preferably, at least 90% sequence identity to SEQ ID NO: 13 (i.e., G9/RecHTLV). The sequence identity can also be at least 95%, at least 96%, at least 97%, at least 98% or at least 99%. In a preferred embodiment, the recombinase comprises SEQ ID NO: 13 (G9/RecHTLV).

Other recombinases of the invention are E5_E9 (also called E5), A3_A9_C6, A11_E1; B11, B12, C2, D7, D11_F7_F9_G8, F6, F11, F12, G7, G10 and H2 (SEQ ID NO: 14 and 26-37, 39).

Preferably, the tailored recombinase of the invention further comprises SEQ ID NO: 38. SEQ ID NO: 38 is a consensus sequence of all recombinase clones of the invention that were sequences. SEQ ID NO: 38 has 343 amino acids and 101 variable positions X. Accordingly, the consensus sequence provides guidance to the skilled person which domains and positions of the recombinase are variable, and which positions should only be mutated with caution. X can be any naturally occurring amino acid. Amino acids in variable positions preferably are conservative substitutions compared to the sequence of any of the clones provided herein, wherein a given amino acid is substituted to a different amino acid with similar biochemical properties (e.g. charge, hydrophobicity and size). For example, aliphatic amino acids, hydroxyl-containing or sulfur-containing amino acids, cyclic amino acids, aromatic amino acids, basic amino acids or acidic amino acids or their amines can be exchanged within these groups.

It is preferred that the recombinase has, in any position, an amino acid that is present in said position in any of G9, E5_E9 (also called E5), A3_A9_C6, A11_E1; B11, B12, C2, D7, D11_F7_F9_G8, F6, F11, F12, G7, G10 and H2 (SEQ ID NO: 13, 14 and 26-37, 39).

Optionally, said tailored recombinase further (in addition to the substitutions recited above) comprises at least one, preferably, 2 to 101 (e.g., 5-90, 10-80, 20-70, 30-60, or 40-50) of the following defined amino acids in the recited positions (wherein the positions are compared to SEQ ID NO: 11 or with reference to SEQ ID NO: 11):
- 3K, 3N, or 3D, preferably, 3K;
- 5L, 5Q or 5P, preferably, 5L;
- 7L or 7I, preferably, 7L;
- 8H, 8P or 8Y, preferably, 8H;
- 9Q or 9H, preferably, 9Q;
- 10S or 10D or 10N, preferably, 10S;
- 12S or 12F or 12T, preferably, 12S;
- 14L or 14S, preferably, 14L;
- 16A or 16V, preferably, 16A;
- 17D or 17G or 17N, preferably, 17D;
- 18A or 18V, preferably, 18V;
- 19T or 19A or 19M, preferably, 19T;
- 23A or 23T, preferably, 23A;
- 25K, 25Q or 25R, preferably, 25K;
- 26N or 26S, preferably, 26N;
- 28M or 28T, preferably, 28M;
- 29D or 29V or 29I, preferably, 29D;
- 30M or 30T or 30V, preferably, 30M;
- 31F or 31L, preferably, 31F;
- 34R or 34H or 34C, preferably, 34R
- 35Q or 35H, preferably, 35Q;
- 39E or 39A, preferably, 39E;
- 43K or 43E or 43R; preferably, 43E;
- 45L or 45F, preferably, 45L;
- 51S or 51A or 51T; preferably, 51S;
- 54A or 54T, preferably, 54A;
- 57 E or 57K, preferably, 57E;
- 58S or 58L, preferably, 58S;
- 59N or 59D, preferably, 59N;
- 62K or 62R, preferably, 62K;
- 63W or 63R, preferably, 63W;
- 68P or 68H, preferably, 68P;
- 77H or 77Y or 77D; preferably, 77H;
- 86N or 86S, preferably, 86N;
- 88V or 88I, preferably, 88V;
- 93A or 93T, preferably, 83A;
- 94E or 94D, preferably, 94E;
- 97T or 97M, preferably, 97T;
- 110S or 110N, preferably, 110S;
- 111N or 111S, preferably, 111N;
- 114T or 114S or 114P, preferably, 114T;
- 125V or 125I, preferably, 125V;
- 132K or 132R, preferably, 132K;
- 136A or 136P, preferably, 136A;
- 140T or 140A, preferably, 140T;
- 144R, 144Q or 144L, preferably, 144R;
- 147S, 147T, 147P or 147A, preferably, 147S;
- 149M or 149L, preferably, 149M;
- 151N, 15 1T, 151D or 151S; preferably, 151N;
- 155C or 155R, preferably, 155C;
- 156L, 156K or 156Q, preferably, 156L;
- 160N or 160D, preferably, 160N;
- 183K or 183R, preferably, 183K;
- 185I or 185V, preferably, 185I;
- 196H or 196R, preferably, 196H;
- 206T or 206A, preferably, 206T;
- 207A or 207T, preferably, 207A;
- 219K, 219R or 219X, preferably, 219K;
- 221V or 2211, preferably, 221V;
- 225I, 225V or 225F, preferably, 225I;
- 226S or 226T, preferably, 226S;
- 227V or 227A, preferably, 227A;
- 229G or 229C, preferably, 229G;
- 230V or 230I, preferably, 230V;
- 231A or 231T, preferably, 231A;
- 232D or 232N, preferably, 232D;
- 235N or 235D, preferably, 235N;
- 241R or 241Q, preferably, 241R;
- 244R or 244K, preferably, 244R;
- 247V or 247I, preferably, 247V;
- 248V or 248A, preferably, 248V;
- 249V or 249A, preferably, 249V;
- 250S or 250P, preferably, 250S;
- 253T or 253A, preferably, 253T;
- 254S, 254K, 254N or 254C, preferably, 254S;
- 255R or 255Q, preferably, 255R;
- 258T or 258A, preferably, 258T;
- 259H, 259Y or 259G, preferably, 259H;
- 261M or 261L, preferably, 261M;
- 262Q or 262R, preferably, 262Q;
- 263G or 263K, preferably, 263G;
- 264I or 264V, preferably, 264I;
- 267A or 267T, preferably, 257A;
- 272I or 272V, preferably, 272I;
- 276K, 276R or 276N, preferably, 276K;
- 277D or 277G, preferably, 277D;
- 278D or 278G, preferably, 278D;
- 280G or 280S, preferably, 280G;
- 281Q, 281R or 281L, preferably, 281Q;
- 299L or 299M, preferably, 299L;
- 304V or 304A, preferably,304V;
- 305S or 305P, preferably, 305S;
- 306I, 306M or 306L, preferably, 306I;
- 307A, 307P or 307V, preferably, 307A;
- 317R or 317T, preferably, 317R;
- 319N or 319D, preferably, 319N;
- 320S, 320T or 320G, preferably, 320S;
- 332T or 332A, preferably, 332T;
- 341D or 341G or 341E, preferably, 341D;
- 342G or 342D, preferably, 342G; and/or
- 343D or 343Y, preferably, 343D.

The amino acids may be selected independently of each other. The amino acids present in the highest activity clone G9 are considered preferred. A recombinase may comprise at least 1, preferably, 2-101 (e.g., 5-90, 10-80, 20-70, 30-60, or 40-50) of said preferred positions. Further, generally, it is preferred that combinations of positions present in active recombinases are present together.

The amino acids analysis of the different recombinases of the invention shows that preferred amino acids position are 94E or 94D, preferably, 94E. These substitutions are also not known in the art.

Further novel combinations of amino acids positions have been identified in positions 247 and 248. Preferably, the tailored recombinase of the invention comprises either
i) 247V and 248V and 244R or
ii) 247I and 248A.

A particularly high activity has been shown for recombinases of the invention comprising 247V and 248V and 244R. These recombinases preferably also comprise 249V.

Preferably, said recombinase further comprises a substitution in position N317, where ere has N, i.e., the amino acid in position 317 is not N. It was found that said position can be, e.g., N317R or N317T. Preferably, it is N317R.

The changes of P12S, P15L, V23A, S38P, Y77H, A84T, K86N, Q90K, G93A, Q94E, M97T, S108G, K122R, I166V, A175S, K244R, N245Y, A248V, A249V, R259H, L261M, E262Q, E266G, T268A, N317R or N317T, and I320S are present in both preferred recombinases E5 and G9/RecHTLV and in more than 75% of the sequenced clones. They are therefore considered to represent the most essential changes. These exchanges render the enzyme particularly suited for recombination at a target sequence of SEQ ID NO: 1 or a target sequence having a high sequence identity to SEQ ID NO: 1 (e.g., at least 80%, at least 90% or at least 95% sequence identity to SEQ ID NO: 1).

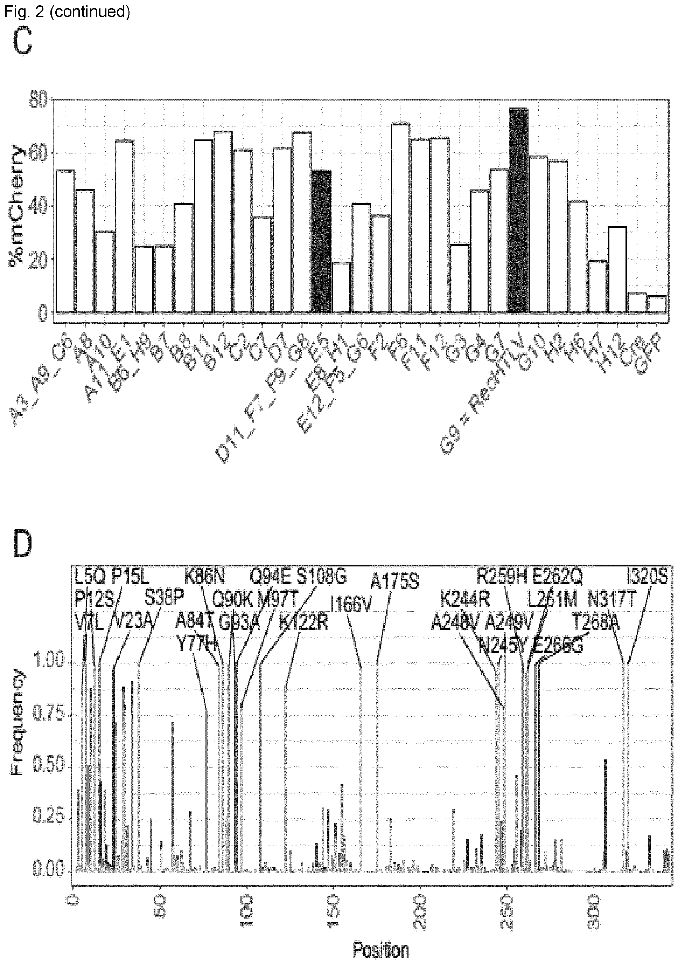

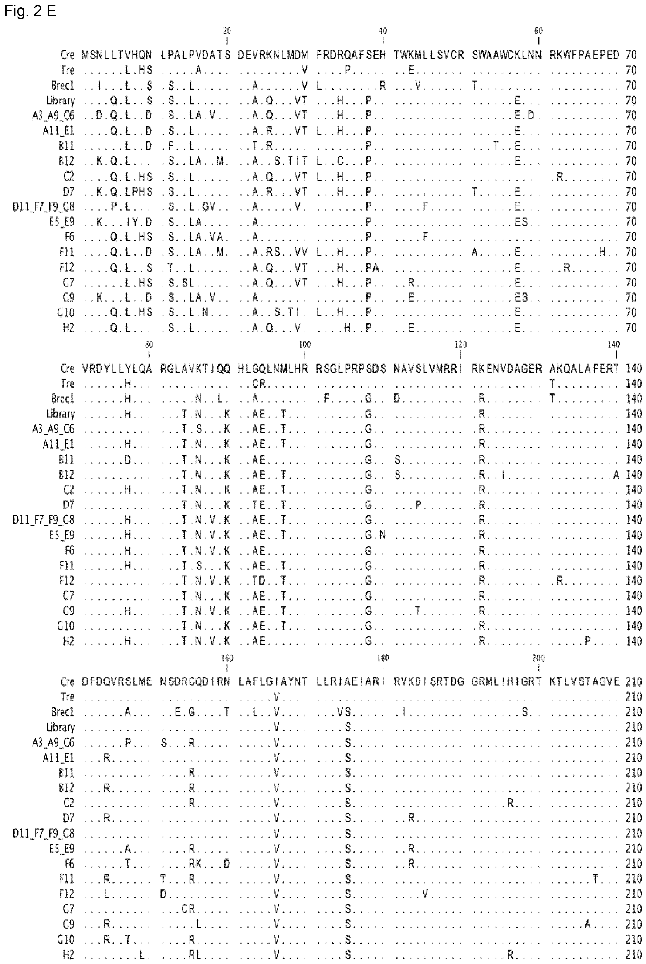

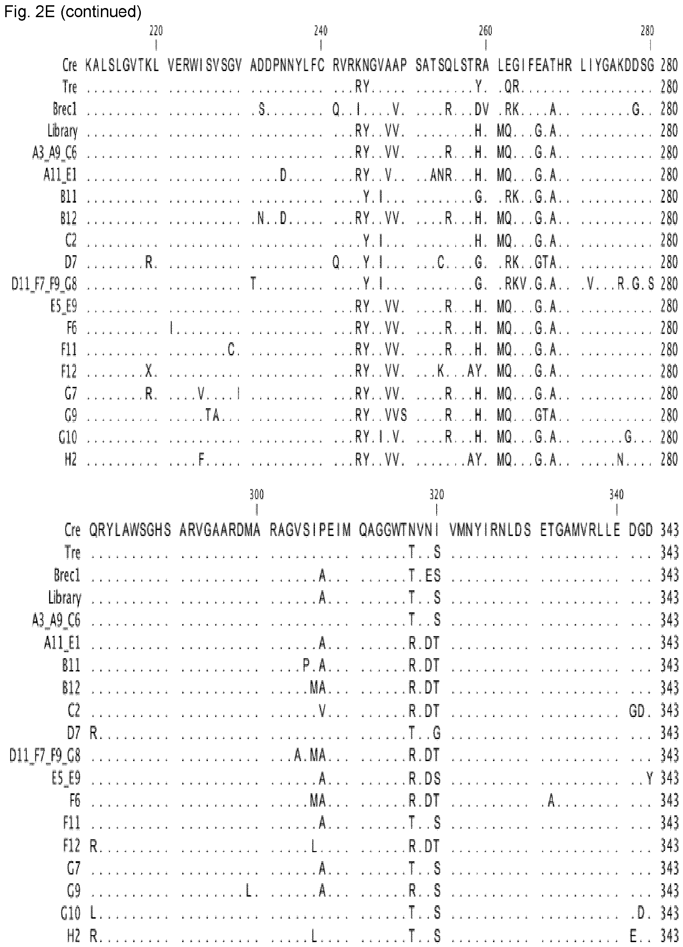

The changes indicated in Fig. 2C, namely, V7L, P12S, P15L, V23A, S38P, Y77H, A84T, K86N, Q90K, G93A, Q94E, M97T, S108G, K122R, I166V, A175S, K244R, N245Y, A248V, A249V, R259H, L261M, E262Q, E266G, T268A, N317T and I320S are present in more than 75% of the sequenced clones. Except for N317T (G9 has N317R), they are also present in the highly active recombinase variant G9/RecHTLV (but not necessarily in E5). A recombinase of the invention (and a nucleic acid encoding it) comprising these amino acid exchanges compared to ere is also provided by the invention.

It is preferred that the nucleic acid of the invention encodes a recombinase comprising at least 33, preferably, all amino acid exchanges compared to SEQ ID NO: 11 selected from the group consisting of N3K, N10D, P12S, P15L, V16A, V23A, S38P, K57E, L58S, Y77H, A84T, K86N, I88V, Q90K, G93A, Q94E, M97T, S108G, K122R, I166V, A175S, K244R, N245Y, A248V, A249V, Q255R, R259H, L261M, E262Q, E266G, T268A, P307A, N317R, and I320S. These amino acid exchanges are present in both E5 and G9/RecHTLV.

Preferably, the encoded tailored recombinase has at least 95%, at least 96%, at least 97%, at least 98%, or at least 99% sequence identity with SEQ ID NO: 12. SEQ ID NO: 12 is the consensus sequence of two advantageous tailored recombinases, E5 and G9/RecHTLV, that are provided by the invention. The recombinase may thus comprise an amino acid sequence according to SEQ ID NO:12.

The nucleic acid of any of the preceding claims, wherein said tailored recombinase comprises at least 34, preferably, all amino acid exchanges compared to SEQ ID NO:11 selected from the group consisting of N3K, V7L, N10D, P12S, P15L, V16A, A18V, V23A, S38P, K43E, K57E, L58S, Y77H, A84T, K86N, I88V, Q90K, G93A, Q94E, M97T, S108G, S114T, K122R, Q144R, Q156L, I166V, A175S, T206A, S226T, V227A, K244R, N245Y, A248V, A249V, P250S, Q255R, R259H, L261M, E262Q, E266G, A267T, T268A, M299L, P307A, N317R, and I320S. All of these substitutions are comprised in RecHTLV.

In a particularly preferred embodiment, the encoded recombinase comprises the amino acid sequence according to SEQ ID NO: 13 (G9/RecHTLV) or 14 (E5), or A3_A9_C6, A11_E1; B11, B12, C2, D7, D1 1_F7_F9_G8, F6, F11, F12, G7, G10 and H2 (SEQ ID NO: 26-37, 39), or a combination of any of said sequences. A combination of said sequences has consensus sequence SEQ ID NO: 38 and consists of at least one part of one of the recited defined sequences, e.g., SEQ ID NO: 13 and at least one part of another of said recited sequences, e.g., SEQ ID NO: 14, e.g., an N-terminal part of one of said sequences (e.g., SEQ ID NO: 13) and a C-terminal part of another of said recited sequences, or vice versa. The tailored recombinase may also be a combination of three or more (e.g., four or five) parts derived from two or more (e.g., three, four or five) of said sequences.

The tailored recombinase may be highly specific for recombination of SEQ ID NO: 1. It may also recombine other sites, e.g., loxP, though.

However, as shown below, the recombinases of the invention advantageously have no detectable activity on human sequences that allow for excision or modification of human sequences from or in the genome. This has the advantage that, when the recombinase of the invention is applied for therapy of humans, the risk of cross-reaction with and recombination of human sequences in the host cell is minimal. This is one factor contributing to tolerability of the recombinase in human cells. However, not only short-term consequences of expression of the recombinase are at issue here, but also safety aspects such as possible oncogenic effects of unspecific recombination, even at low efficacy. Elimination of even residual activity of the tailored recombinase thus contributes to safety and reliability of the resulting recombinase in therapeutic settings.

In the context of the invention, a nucleic acid or a protein comprising a sequence may consist of said sequence.

It may alternatively comprise further sequences, i.e., it may be a fusion protein. Such further sequences may be, e.g., a signal sequence providing for expression / localization in a specific cellular compartment such as a nuclear localization signal, as in SEQ ID NO:25, or nucleic acid sequences encoding such a signal.

If a protein is to be used in a pharmaceutical composition, it is especially preferred to express it as a fusion protein with a protein transduction domain such as the tat protein transduction domain, which allows for protein transduction of target cells.

Preferably, a recombinase of the invention which is to be used in a pharmaceutical composition is prepared as a fusion protein with a nuclear localization sequence and with a protein transduction domain e.g. from tat, and a nucleic acid encoding a recombinase of the invention may encode such a fusion protein. For example, the following protein transduction domains may be used in a fusion protein with a recombinase of the invention, which preferably further includes a nuclear localization signal:
- Basic domain of HIV- 1 Tat transactivator (Fawell *et al,* 1994) (Fawell S, Seery J, Daikh Y, Moore C, Chen LL, Pepinsky B, Barsoum J., Tat-mediated delivery of heterologous proteins into cells., Proc Natl Acad Sci U S A. 1994 Jan 18;91(2):664-8.)
- Homeodomain of Drosohila antennapedia (Antp) (Derossi *et al,* 1994) (Derossi D, Joliot AH, Chassaing G, Pro- chiantz A., The third helix of the Antennapedia homeodomain translocates through biological membranes., J Biol Chem. 1994 Apr 8;269(14): 10444-50.)
- HSV VP22 transcription factor (Elliott & O'Hare, 1997) (Elliott G, O'Hare P., Intercellular trafficking and protein delivery by a herpesvirus structural protein., Cell. 1997 Jan 24;88(2):223-33.)
- Cell permeable translocation motif (TLM) of PreS2 surface antigen of Hepatitis B virus (HBV) (Oess & Hildt, 2000) (Oess S, Hildt E., Novel cell permeable motif derived from the PreS2-domain of hepatitis-B virus surface antigens., Gene Ther. 2000 May;7(9):750-8.).

In case the protein is to be purified, a tag facilitating purification of a protein such as a His tag, may also be added.

Fusion proteins may be prepared by methods well known in the art. For example, the expression vector into which the nucleic acid encoding the tailored recombinase is cloned may already comprise a nucleotide sequence encoding a second polypeptide or protein. By cloning the nucleic acid encoding the tailored recombinase in frame with the sequence of the second polypeptide or protein, both sequences will be expressed as fusion protein.

The codon usage of the nucleic acid of the invention encoding a recombinase as defined above can be chosen by the skilled person. For example, a codon usage suitable for expression in a human cell may be chosen, in particular, if expression in a human cell is intended, e.g., for therapeutical purposes. Codon usage may also be based on codon usage of e.g., Cre recombinase.

The recombinase or nucleic acid encoding said recombinase may be obtained by the method of the invention as described herein, or it may be obtainable by this method. It may also be obtained based on the sequences disclosed herein, optionally, by combining and/or further varying these sequences, optionally testing for activity in recombination of asymmetric target sites, such as SEQ ID NO: 1.

The invention thus provides a nucleic acid encoding a tailored recombinase capable of recombining asymmetric target sequences such as SEQ ID NO: 1 within the LTR of proviral DNA of the majority of HTLV-1 strains which may be inserted into the genome of a host cell, the tailored recombinase comprising an amino acid sequence as defined above.

In the method of the present invention, the nucleic acid encoding a tailored recombinase that is active on the asymmetric target sequence within the LTR of the retroviral DNA is preferably cloned into an expression vector. Thus, the present invention is also directed to an expression vector comprising the nucleic acid encoding a tailored recombinase as defined herein.

Expression vectors are genetic constructs for expressing the proteins encoded by the nucleic acids within the vector. Such expression vectors may be either self-replicating extrachromosomal vectors or vectors which integrate into a host genome. Generally, these expression vectors include a transcriptional and translational regulatory nucleic acid operably linked to the nucleic acid encoding the tailored recombinase of the present invention.

A "nucleic acid" as used herein is a polymeric compound comprised of covalently linked sub- units called nucleotides. Nucleic acid includes polyribonucleic acid (RNA), e.g., mRNA, and polydeoxyri-bonucleic acid (DNA), both of which may be single-stranded or double-stranded. DNA includes cDNA, genomic DNA, synthetic DNA, and semi-synthetic DNA.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

A nucleic acid is "operably linked" when it is placed into a functional relationship with another nucleic acid sequence. For example, a promoter or enhancer is operably linked to a coding sequence if it affects the transcription of the sequence; or a ribosome binding site is operably linked to a coding sequence if it is positioned so as to facilitate translation. Linking is accomplished by ligation at convenient restriction sites. If such sites do not exist, synthetic oligonucleotide adaptors or linkers are used in accordance with conventional practice. The transcriptional and translational regulatory nucleic acid will generally be appropriate to the host cell used to express the tailored recombinase. Numerous types of appropriate expression vectors, and suitable regulatory sequences are known in the art for a variety of host cells.

The expression vector used in the present invention may be a retroviral vector, a lentiviral vector, a spumavirus vector, an adenoviral vector, or an adeno-associated virus vector. It may also be a transposon such as a transposon capable of being transposed by Sleeping Beauty, e.g., SB100X or a variant thereof. An alternative transposon can be transposed by Frog Prince. However, in a preferred embodiment, the expression vector is a lentiviral vector selected from the group consisting of HIV- 1 -, SIV-, FIV- or EIAV-derived lentiviral vectors. Lentiviral vectors are for example described by Schambach *et al.* (2006) or in European Patent Application No. 1 1000751.5.

In preferred embodiments of the present invention the expression vector comprises a cellular, bacterial, a viral or a hybrid promoter.

In general, for the purpose of the present invention, the promoter may be a constitutive or an inducible promoter. Further, the promoter may be either a naturally occurring promoter, such as a bacterial, cellular or a viral promoter, or a hybrid promoter. Hybrid promoters, which combine elements of more than one promoter, are known in the art, and are useful in the present invention. Further, the promoter used in the present invention may also be a derivative of a naturally occurring promoter. A "derivative" of a naturally occurring promoter as used herein may be a combination of cis-active elements obtained from promoters or sequences of different origin or, alternatively, may be obtained by deletion or mutation of cis-active elements within a specific naturally occurring promoter (e.g., as taught by Edelmann et al, 2000; Hartenbach & Fussenegger, 2006; Alper *et al,* 2005).

In an embodiment of the present invention, the constitutive promoter or derivative thereof is selected or derived from the group consisting of promoters of cytomegalovirus, Rous sarcoma virus, murine leukemia virus-related retroviruses, phosphoglycerokinase gene, murine spleen focus-forming virus or human elongation factor 1 alpha.

In a further embodiment of the present invention, the inducible promoter or derivative thereof is selected or derived from the group consisting of the LTR or derivatives thereof derived from lentiviruses, spumaviruses and deltaretroviruses.

In this context, the term "LTR" refers to both the 5' and the 3' long terminal repeats of provirus having promoter function (for a review see the standard textbook "Retroviruses" (Coffin et al, 1997)).

Preferably, the inducible promoter or derivative thereof is selected or derived from the LTR or derivatives thereof derived from HIV-1, HIV-2, MVV, EIAV, CAEV, SIV, FIV, BIV, HTLV- I and HTLV-II.

The present invention further provides a method for preparing a tailored recombinase, wherein said method comprises a method for preparing an expression vector encoding a tailored recombinase of the invention, and the further step of expressing said tailored recombinase (or a fusion polypeptide comprising the amino acid sequence of said tailored recombinase) from said expression vector in a suitable host cell.

Preferably, the recombinases finally obtained are tested in mammalian cells to ensure that they function in a mammalian cell environment. Further, to obtain good expression in mammalian cells the recombinases may be optimized for expression in these cells (e.g. codon usage optimization using methods well known in the art. See for example (Shimshek *et al,* 2002) or signal sequences necessary for directing the protein into the nucleus of the mammalian cell, such as the NLS sequence (Macara, 2001) may be added to the nucleic acid of the tailored recombinase. Expression of the nucleic acid encoding the tailored recombinase cloned into an expression vector can be carried out using for example bacterial, insect or mammalian expression systems. However, other expression systems known in the art may also be employed. Methods of introducing exogenous nucleic acid into mammalian, insect or bacterial hosts, as well as other hosts, are also well known in the art, and will vary with the host cell used. Techniques include dextran-mediated transfection, calcium phosphate precipitation, polybrene mediated transfection, protoplast fusion, electroporation, viral infection, encapsulation of the polynucleotide(s) in liposomes, and direct microinjection of the DNA into nuclei.

The invention thus also provides a tailored recombinase encoded by the nucleic acid of the invention, i.e., a protein. The term "protein" as used herein includes proteins, polypeptides, and peptides. As will be appreciated by those in the art, the nucleic acid sequences of the invention can be translated into protein sequences. The recombinase is optionally a fusion protein. In one embodiment, the tailored recombinase protein is prepared as a fusion polypeptide to increase expression. In a further embodiment, the tailored recombinase protein is made as a fusion polypeptide to allow introduction of the polypeptide into living cells. Typically, purified proteins cannot enter into cells, because they are not able to pass the cell membrane due to their size. However, fusion of specific peptides sequences to proteins can result in the uptake of these fusion proteins into cells. In the cell the protein can then perform its function. Site specific recombinases, including Cre recombinase, have been successfully delivered into cells with this approach (Peitz *et al,* 2002). Cell-permeant recombinases have further been described by (Nolden *et al,* 2006) and (Lin *et al,* 2004). Hence, this strategy may be used to deliver the tailored recombinases into cells to remove the provirus from infected cells. Thus, the second polypeptide in the fusion polypeptide may comprise a signal peptide. The signal peptide may be a protein transduction domain such as the TAT peptide or a peptide from the third helix of the Antennapedia homeodomain (Derossi *et al,* 1996, 1994; Vivès *et al,* 1997, 2003; Richard *et al,* 2005) or the NLS (nucleus localization sequence) for delivering the fusion polypeptide into the nucleus of an eukaryotic cell (Macara, 2001). For example, a fusion protein of the invention may comprise a NLS such as SEQ ID NO: 25, tat and a recombinase sequence of the invention such as RecHTLV or E5.

In a further embodiment, the invention provides a transformed cell comprising the nucleic acid of the invention. Transformed cells used for expressing the tailored recombinase from an expression vector include prokaryotic cells, such as for example bacterial cells or yeast cells, or, preferably, eukaryotic cells, such as for example insect cells or mammalian, most preferably, human cells. The host cell may be a hematopoietic cell, e.g., an adult hematopoietic stem cell or a T-cell, e.g., a CD4+ cell. The cell may be derived from a subject infected with the retrovirus, and the cell may be administered back to the subject after transformation, and, optionally, cultivation and/or propagation.

The transformed cell preferably is, at least in the context of therapy of a human subject, a human cell, e.g., a human stem cell. Said cell preferably is a stem cell from the hematopoietic lineage, e.g., a CD34+ stem cell. Stem cells may be adult stem cells, embryonic stem cells, or induced pluripotent stem cells. In the state of the art, the term "stem cells" designates cells which (a) have the capability of self-renewal and (b) the capability to form at least one and often a number of specialized cell types due to their asymmetrical division capability (Donovan & Gearhart, 2001).

The stem cells are preferably infected or transfected with the expression vector according to the invention. In a preferred embodiment, the stem cell is a stem cell from the hematopoietic lineage expressing the tailored recombinase, the aforementioned fusion polypeptide or comprising the aforementioned expression vector. Hematopoietic stem cells (HSC) are bone marrow-derived CD34+ cells, which can, e.g., be purified from G-CSF-mobilized peripheral blood of donors (e.g., HIV-infected patients) by routine leukapheresis (Scherr & Eder, 2002). The in vitro genetically modified cells may then be formulated for reinfusion into the patients.

In a further embodiment, the expression vector of the present invention is used for transforming T-cells, e.g., CD4+ primary cells (blood cells) of HTLV-1-infected patients. A cell line such as Jurkat T cells can also be employed.

Alternatively, the tailored recombinase of the invention may be formulated for delivery by virus-like particles (VLPs). VLPs can be used to package recombinase mRNA, recombinase protein, e.g., fusion protein, or DNA, e.g., recombinase expressing DNA plasmids, or a construct comprising Promotorrecombinase cDNA-polyA site. Accordingly, the nucleic acid of the invention may further contain a packaging signal.

The inventors have for the first time identified the target sequence of SEQ ID NO: 1 in HTLV-1 that can advantageously be used for excision of a HTLV-1 provirus comprising said sequence, which is the case for the majority of strains.

The invention also provides a pharmaceutical composition comprising a nucleic acid of the invention, a tailored recombinase of the invention, and/or a transformed cell the invention. The pharmaceutical composition optionally comprises a pharmaceutically acceptable excipient, adjuvant and/or solvent. Solvents or carriers, excipients and adjuvants suitable for use in a pharmaceutical composition are known in the art. The pharmaceutical composition is preferably present in the form of a solution suitable for intravenous application (infusion).

According to the invention, the nucleic acid of the invention, the expression vector comprising the nucleic acid encoding a tailored recombinase of the invention, the recombinase protein, the fusion protein or the cell, e.g., stem cell of the invention or obtained by the methods of the present invention are formulated as a pharmaceutical composition. Said composition may be for use in prevention and/or treatment of a HTLV-1infection and/or for the reduction of the viral load in a subject infected by HTLV-1. A further object of the invention is the pharmaceutical composition obtained by the method of the invention as described herein.

The pharmaceutical composition of the present invention comprising an expression vector encoding a tailored recombinase (or the tailored recombinase as a protein or fusion polypeptide or a stem cell comprising the expression vector) is capable of reducing the virus load in a subject infected with HTLV-1 by eradicating the genetic reservoir of said virus within hosts cells, thereby preventing further life cycles of the virus.

The subject infected by HTLV-1 retrovirus, to whom the pharmaceutical composition is to be administered, is selected from the group consisting of humans, primates, monkeys, cattle, horses, goats, sheep and domestic cats. However, the subject is preferably a human being.

In general, an effective amount of the expression vector, the tailored recombinase or the transformed cell of the invention is to be administered to the subject. Administration may be, e.g., intravenous or intramuscular administration.

For example, the pharmaceutical composition may comprise a transformed cell comprising a nucleic acid of the invention that is capable of expressing a tailored recombinase of the invention. The transformed cell may, e.g., be a human hematopoietic stem cell.

In one embodiment, the pharmaceutical composition is formulated for concomitant administration with antiretroviral agents or concomitant or subsequent to global immune activation therapy or specific activation of provirus gene expression. Global immune activation (activation of immune cells, including resting cells) is usually achieved by, for example, administration of immunotoxins, cytokines (e.g., IL-2), or T cell activating antibodies (e.g., OKT3). Global immune activation therapy or specific activation of provirus gene expression or similar therapy strategies greatly benefits from the concurrent removal of proviral DNA, thereby reducing in the patient the pool of infected cells.

The pharmaceutical composition of the invention preferably is for use in, typically, treatment or, alternatively, prevention of a HTLV-1 infection in a subject, wherein the composition optionally is for use in treatment of adult T-cell leukemia (ATL) and/or HTLV-1-associated myelopathy (HAM). Prevention is understood to be reduction of the likelihood of infection or re-infection.

Optionally, the pharmaceutical composition is formulated for administration to a subject, if proviral DNA found in a sample obtained from the subject comprises the asymmetric target sequence of SEQ ID NO: 1.

The present invention also provides a method of treatment and/or prevention of a HTLV-1 infection in a subject comprising administering an effective amount of a pharmaceutical composition of the invention to the subject. In one embodiment, the sequence of the HTLV-1 infecting the subject is analyzed in a sample obtained from the subject, and at least one expression vector encoding a tailored recombinase, at least one tailored recombinase or at least one cell transformed with said expression vector, e.g., an stem cell, is to be administered to the subject, if the proviral DNA from the subject comprises the asymmetric target sequence of SEQ ID NO: 1 on which the recombinase has been selected. The sample obtained from the subject may be a blood sample, e.g., comprising infected CD4+ cells.

Prevention of a disease is understood to mean reduction of the incidence of the disease.

Also disclosed is a method for preparing a nucleic acid or an expression vector encoding a tailored recombinase capable of recombining asymmetric target sequences within the long terminal repeat of proviral DNA of a plurality of HTLV-1 strains, comprising steps of providing a target sequence present in the LTR of a plurality of proviral DNA of HTLV-1 strains, having a homology (i.e., sequence identity) of at least 30% to the left half-site sequence and the right half-site sequence of at least one known recombinase target site in the sequence of the LTR of proviral DNA of a plurality of HTLV-1 strains, wherein the homologous sequences are separated by a spacer of 5-12 nucleotides, wherein said target sequence preferably is SEQ ID NO: 1; and generating, through repeated steps of
i) molecular directed evolution on at least one recombinase recognizing the known homologous target site using as substrate modified target sequences based on the sequence of the asymmetric target sequence, but modified to contain only a limited number of variations from the known target sequence; wherein, in each round, the target sequence may vary from the target sequence on which the recombinase is known to act in one, two or three nucleotides; and
ii) shuffling the recombinase libraries to obtain recombinase libraries able to recombine target sequences more homologous to the asymmetric target sequence;

until at least one recombinase is obtained that is active on the asymmetric target sequence within the LTR of the HTLV-1 DNA;
repeated steps of negatively selecting against recombination of the known target site by molecular directed evolution and shuffling of the libraries;
selecting said tailored recombinase or recombinases by expressing the library in human cells, in particular, in HeLa cells or human T cells, and culturing said human cells expressing the tailored recombinase(s) for at least 1 week, preferably, at least 2 weeks, and isolating the nucleic acid(s) of the recombinase(s) from the cultured cells expressing the selectable marker;
and, optionally, cloning the nucleic acid encoding the recombinase(s) into a suitable expression vector.

The present invention for the first time provides, with SEQ ID NO: 1, a target sequence in HTLV-1 provirus LTRs that is suitable for excision of the provirus in a high percentage of HTLV-1 strains. Recombinases have been tailored to recognize asymmetric target sites different from their native symmetric target sites, which may be present in a plurality of retrovirus strains, by splitting up the substrate into a number of new subsets with smaller differences from the original target and stepwise tailoring recombinases to recognize these subsets (e.g., WO 2008/083931 and WO 2011/147590). A combinatorial approach allows selection of functional molecules recognizing the asymmetric target site within a given sequence. Thus, traversing through substrate intermediates during directed molecular evolution, it has been possible to produce enzymes with remote novel asymmetric target specificities. This approach is also employed by the present invention. The present invention employs the methods taught by WO 2008/083931 and WO 2011/147590, to target HTLV-1, specifically, SEQ ID NO: 1.

"At least one recombinase" refers to the fact that the method of the invention might lead to one or more (single) recombinases which are, each for itself, active in recombining the asymmetric target sequence. It is not intended to encompass several different recombinases which only together are capable of recombining the asymmetric target sequence. In fact, the method employed for the invention would not lead to selection of recombinases which need to be combined with other, different recombinases to recombine an asymmetric target sequence, because only one recombinase per individual cell is expressed.

The tailored recombinase obtained by the method of the invention is capable of recombining asymmetric target sequences within the LTR of proviral DNA of a HTLV-1. The proviral DNA targeted by the recombinase may be inserted into the genome of a host cell. Alternatively, the tailored recombinase of the invention may recombine asymmetric target sequences within the LTR of HTLV-1 which are not (yet) integrated into the genome of a host cell, i.e. which is present as a non-integrated pre-integration complex (PIC). Thus, HTLV-1 which has not yet integrated into the genome of the host cell as well as HTLV-1 which already has integrated can be inactivated by the tailored recombinase of the invention.

It is to be noted that in the present invention and also in the art the terms "target sequence", "target site" and "recombination site" are used interchangeably.

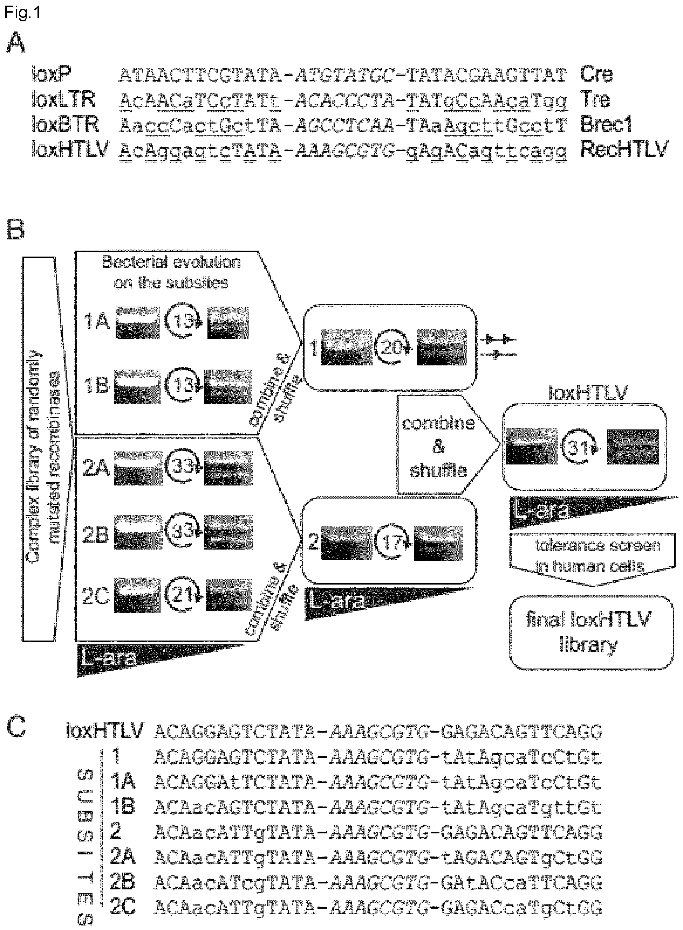

As explained herein, for HTLV-1, a suitable asymmetric target sequence was determined, having the sequence set forth as SEQ ID NO:1 (cf. Fig. 1A, loxHTLV). This renders it possible to generate recombinases, which are practically useful as therapeutic agents against retroviral genomes in a significant number of patients, as these recombinases target recognition sites present across as many strains of the retrovirus as possible.

SEQ ID NO: 1 is present in 86% of the HTLV-1 strains from the HTLV-1 Molecular Epidemiology Database.

The invention also discloses a method for preparing a tailored recombinase wherein said method comprises expressing the tailored recombinase from the nucleic acid encoding the recombinase inserted into the expression vector in a suitable host cell, wherein the recombinase is optionally expressed as a fusion polypeptide comprising the amino acid sequence of the tailored recombinase. The cell optionally is an adult stem cell.

The invention also discloses a method for preparing a transformed cell, wherein said method comprises the method of preparing an expression vector comprising a nucleic acid sequence encoding the recombinase of the invention and the further step of introducing the expression vector into a cell *in vitro.* The cell optionally is an adult stem cell.

### Brief description of the figures

**Fig. 1** A) Alignment of the loxHTLV (RecHTLV) target sequence (SEQ ID NO: 1) to loxP (Cre), loxLTR (Tre) and loxBTR (Bred) (SEQ ID NO: 9, 21 and 22, respectively). Mismatches to the loxP sequence are marked by lowercase and asymmetries within the sequences are underlined. The spacers are shown in italics. B) Scheme of the RecHTLV evolution process. The activity of the recombinase libraries from the first and last cycle in each subsite are shown as images of the agarose gels, where the upper bands correspond to no recombination (two triangles) and the lower bands indicate recombination (one triangle). The number of evolution cycles performed in each subsite is marked between the two agarose gels. The triangles below indicate the reduction of L-arabinose (L-ara) levels during evolution for each target site. C) Alignment of the loxHTLV (RecHTLV) target sequence (SEQ ID NO: 1) to the subsites of evolution (1, 1A, 1B, 2, 2A, 2B, 2C (SEQ ID NO: 2-8). Mismatches compared to loxHTLV are marked by lowercase and spacers are shown in italics.

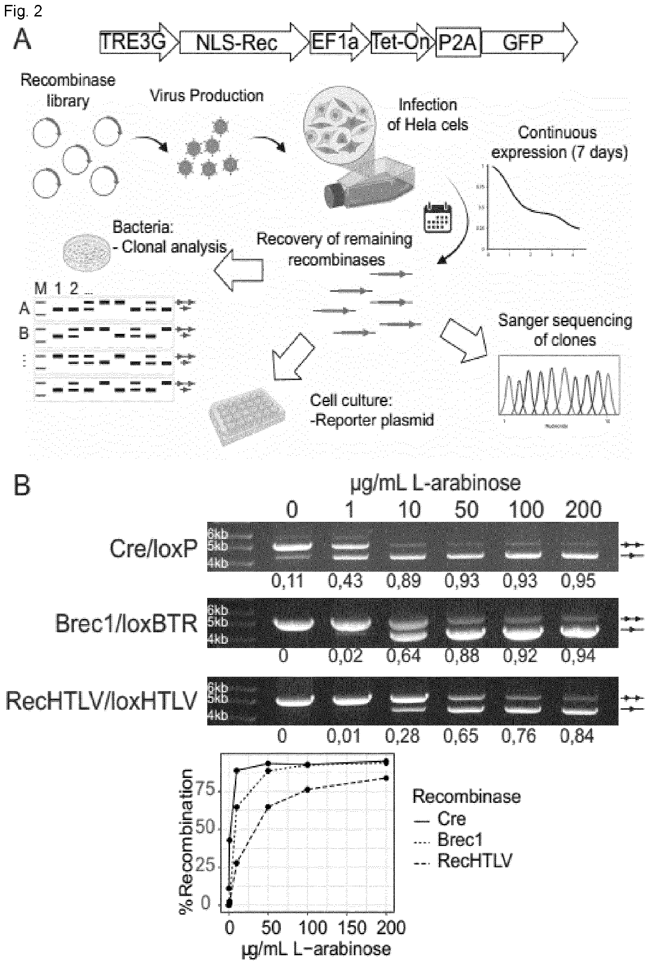

**Fig. 2 A)** Overview of the process for the selection of clones. In the upper part, a scheme of the construct used for expression of the library is shown: expression of the recombinase fused to a nuclear localization signal (NLS-Rec) is regulated by a tetracycline inducible promoter (TRE3G) which is bound by Tet-On 3G (Tet-On Advanced transactivator protein) upon doxycycline treatment; bicistronic expression of Tet-On 3G and a GFP cassette is driven by an elongation factor 1 alpha (EF1a) promoter and separated by a self-cleaving 2A peptide (P2A). The lower part shows the process followed for selection of candidate clones: The recombinase library was cloned into a lentiviral vector and, after lentivirus production, HeLa cells were infected with the recombinase-containing lentiviral particles. After 7 days of expression, the recombinases were extracted by PCR and cloned in the pEVO bacterial plasmid for sequencing and evaluation of activity on the loxHTLV. Then, the clones were tested in the cell culture for activity on the loxHTLV target site.
   **B)** Top: Agarose gels for the plasmid activity assay showing recombination efficiency of Cre, Brec1 and RecHTLV on their respective target sites in bacteria. The performed test digest results in a smaller fragment for recombined (one triangle) and a larger fragment for non-recombined plasmids (two triangles). The recombinases were tested at 0, 1, 10, 50, 100, 200 µg/mL L-arabinose as shown above. Quantification of recombination is shown below the gels as a fraction of 1 (1 fully recombined, 0 not recombined). Bottom: Line graph showing the quantification of recombination at the different levels of arabinose for Cre, Brec1 and RecHTLV. Parts of the figure were created with BioRender.com.
   **C)** Quantification of the recombination activity based on flow cytometry analysis and quantitation of the frequency of mCherry positive cells among the total number of transfected cells (GFP+). The RecHTLV clone G9 has the highest recombination activity is indicated
   **D)** Graph showing the frequency of mutated sequences in the library compared to Cre. Each bar represents an amino acid position. The indicated changes, namely, V7L, P12S, P15L, V23A, S38P, Y77H, A84T, K86N, Q90K, G93A, Q94E, M97T, S108G, K122R, I166V, A175S, K244R, N245Y, A248V, A249V, R259H, L261M, E262Q, E266G, T268A, N317T and I320S are present in more than 75% of the Library and also present in RecHTLV.
   **E)** Alignment of different recombinases Cre, Tre, BREC1, Library, A3_A9_C6, A11_E1; B11, B12, C2, D7, D11_F7_F9_G8, E5_E9 (also called E5), F6, F11, F12, G7, G9 (RecHTLV), G10 and H2 (SEQ ID NO: 11, 15, 16, 24, 14, 13 and 26-37, 39).

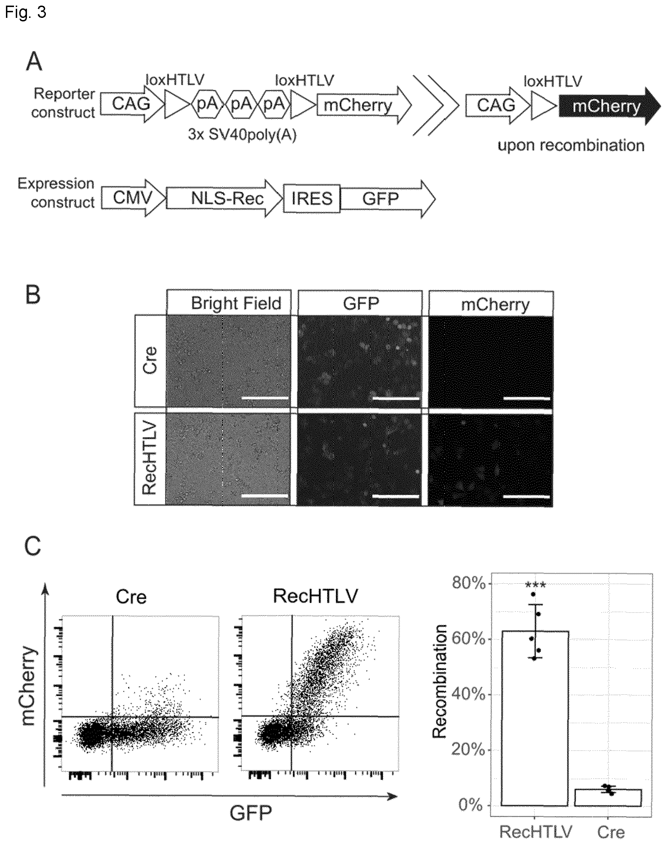

**Fig. 3** A) Schematic representation of the reporter and expression constructs used for assessing RecHTLV activity in HeLa cells. The reporter construct pCAGGS-lox-pA-lox-mCherry consists of 3 poly A (pA) sequences flanked by two loxHTLV sites which prevent the expression of mCherry driven by a CAG promoter. Expression of mCherry is only possible upon recombination of the loxHTLV sites. The IRES (internal ribosomal entry site) bicistronic expression construct allows simultaneous expression of the recombinase fused to a nuclear localization signal (NLS-Rec) and a GFP cassette driven by a CMV promoter. B) Representative images showing the fluorescence-based recombination reporter assay. HeLa cells were co-transfected with the indicated recombinase and the reporter construct. Only upon the expression of RecHTLV we observed expression of mCherry. Scale bar represents 200 µm. First, cells were gated for doublet discrimination and the single cells were then gated based on transfection efficiency (GFP+). From the transfected population mCherry+ cells were quantified. C) Left: representative dot plots obtained by flow cytometry of HeLa cells transfected with the loxHTLV reporter construct and Cre or RecHTLV expression constructs. On the right side, quantification of the recombination efficiency measured as the ratio of mCherry+ cells and frequency of transfected cells (GFP+). The bar shows the mean ± s.d. of five independent experiments (^{∗∗∗} p<0.001, unpaired two-sided t-test).

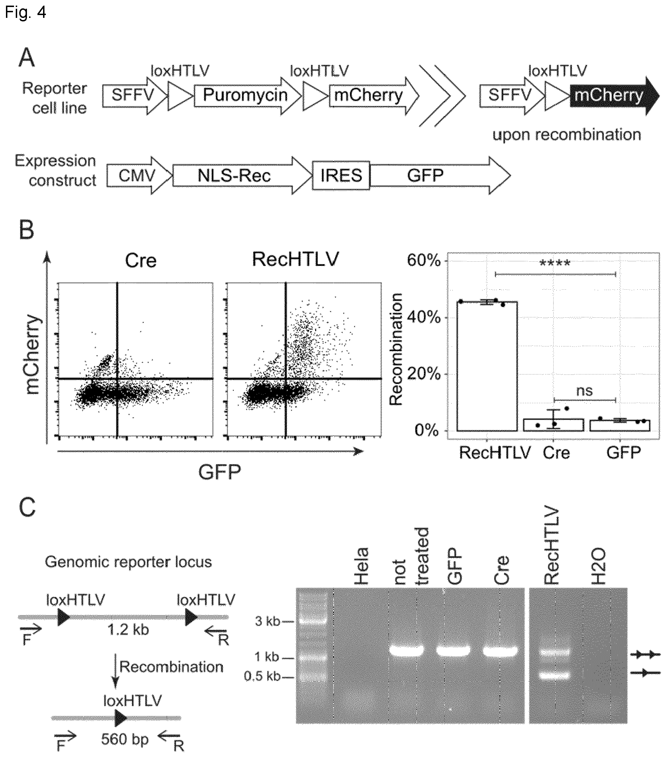

**Fig. 4** A) Schematic representation of the reporter cell line and expression constructs used for assessing RecHTLV activity in HeLa cells in a genomic context. The reporter construct consists of a puromycin cassette flanked by two loxHTLV sites which prevent the expression of mCherry driven by a SFFV promoter. Expression of mCherry is only possible upon recombination of the loxHTLV sites. In the bicistronic recombinase expression construct, the IRES (internal ribosomal entry site) allows simultaneous expression of the recombinase fused to a nuclear localization signal (NLS-Rec) and a GFP cassette driven by a CMV promoter. B) Left: representative dot plots obtained by flow cytometry of the loxHTLV reporter cells transfected with Cre or RecHTLV expression constructs. On the right side, quantification of the recombination efficiency measured as the ratio of mCherry+ cells and the total transfected cells (GFP+). The bar shows the mean ± s.d. of three independent experiments (^{∗∗∗∗}p<0.0001, ns: not significant, unpaired two-sided t-test). C) PCR performed on genomic DNA from transfected loxHTLV HeLa reporter cells. Left: schematic representation of the PCR where primers F and R are located in the flanking regions outside loxHTLV. The expected amplification product is 1.2kb for non-recombined and 560 bp for the recombined reporter. Right: agarose of the PCR products from genomic DNA extracted from different samples: lane 1: HeLa cells, lane2-4: Reporter HeLa cells transfected with the indicated construct, lane 5: water control. The upper band on the gel shows unrecombined reporter (two triangles) while the lower one shows recombination (one triangle).

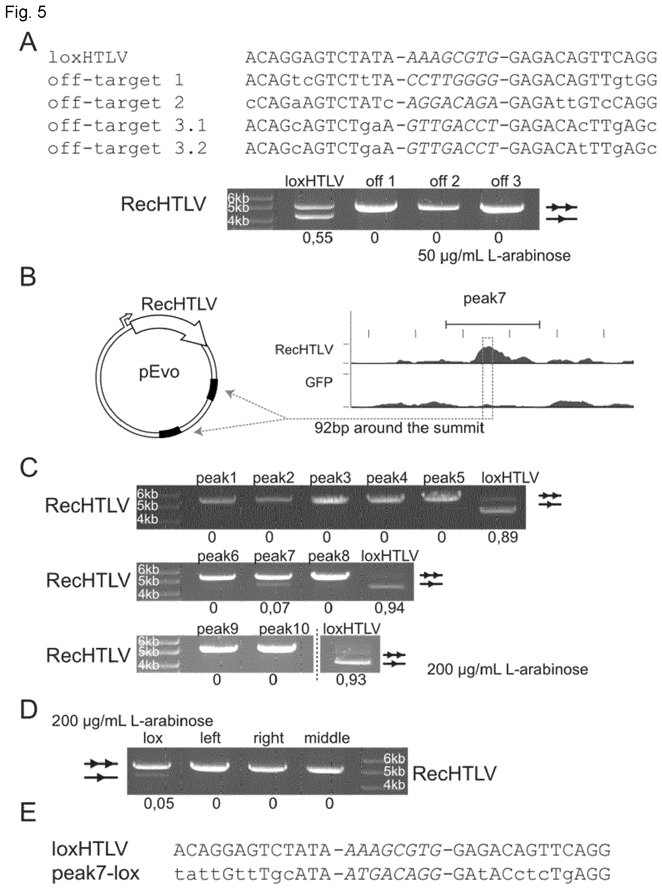

**Fig. 5** A) Top: Alignment of the loxHTLV target sequence to the *in silico* predicted off-target sites: 1, 2 and 3 (SEQ ID NO: 17-20). Off-target-site 3 is asymmetric and is therefore shown as 3.1 and 3.2 in the alignment. Mismatches to the loxHTLV sequence are marked in red and spacers are shown in grey. Bottom: Agarose gel showing the recombination activity of RecHTLV at 50 µg/mL L-arabinose for the on-target sequence (loxHTLV) and the 3 predicted off-targets in bacteria. Off-target sites 1 and 2 were inserted twice as excision substrate in the pEVO vector, whereas off-target site 3 consists of the 3.1 and the 3.2 sequences inserted as excision substrate. Below each lane, quantification of the recombination efficiency is shown. The upper unrecombined band is indicated with two triangles and the recombined band with one triangle. B) Schematic representation of the plasmids used for testing the selected peaks from the ChIP-seq experiment in bacteria. From the selected peaks, 92 bp around the summit of the peak were cloned twice in the pEVO vector as excision substrate (shown for peak 7, chr. 12). C) Bacterial plasmid-based for RecHTLV activity assay of the 10 peaks selected from the ChIP-seq binding sites. All peaks were tested at 200 µg/mL of L-arabinose. Quantification of the recombination is shown below each lane. D) Dissecting the peak 7 to find the RecHTLV recombinase recognition sequence. The sequence of the 92 bp tested in the plasmid assay (SEQ ID NO: 10) was divided into the indicated subdivisions tested: "left" (position 1-46), "middle" (24-70), "right" (47-92), "lox" (40-73P). An agarose gel from the bacterial test digest assay showing the recombination efficiencies of the different parts of peak7 is shown. Only the lox-peak7 sequence (SEQ ID NO: 23) shows recombination at 200 µg/mL L-arabinose. Quantification of the recombination is shown below each lane. The upper unrecombined band is indicated with two triangles and the recombined band with one triangle. E) Alignment of the loxHTLV sequence to peak7-lox. Mismatches to the loxHTLV sequence are marked in red and the spacers are shown in grey.

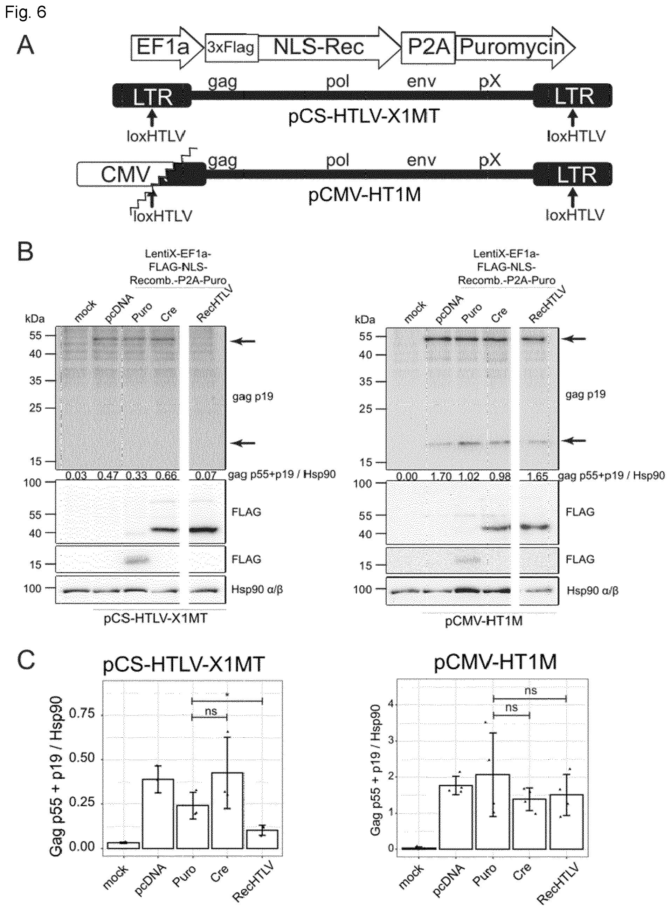

**Fig. 6** A) Scheme of the recombinase expression vector and the HTLV-1 proviral expression plasmids pCS-HTLV-X1MT and pCMV-HT1M. pCS-HTLV-X1MT contains two full-length LTR while pCMV-HT1M has only one truncated 5'LTR, therefore lacking one loxHTLV target site. In the bicistronic recombinase expression construct, the self-cleaving 2A peptide (P2A) allows simultaneous expression of the recombinase fused to a 3xFLAG peptide and a nuclear localization signal (NLS-Rec) and a GFP cassette driven by an elongation factor 1 alpha promoter (EF1a). B) Detection of Gag p55/p19 protein and FLAG-tagged recombinases 72 hours after transient transfection of 293T cells with recombinase expression constructs Puro (control), Cre, and RecHTLV together with proviral expression plasmids (pCS-HTLV-X1MT, pCMV-HT1M). Hsp 90 served as control. Numbers indicate densitometric analysis of Gag (p55 + p19) protein, normalized to Hsp 90. Marked with red arrows are the expected sizes of p55 and p19 products. Blots were cut due to technical reasons. C) Densitometric analysis of Gag protein normalized to Hsp90. The bar shows the mean ± s.d. of three-four independent experiments (* p<0.05, ns: not significant, unpaired two-sided t-test using the logarithm of the normalized values).

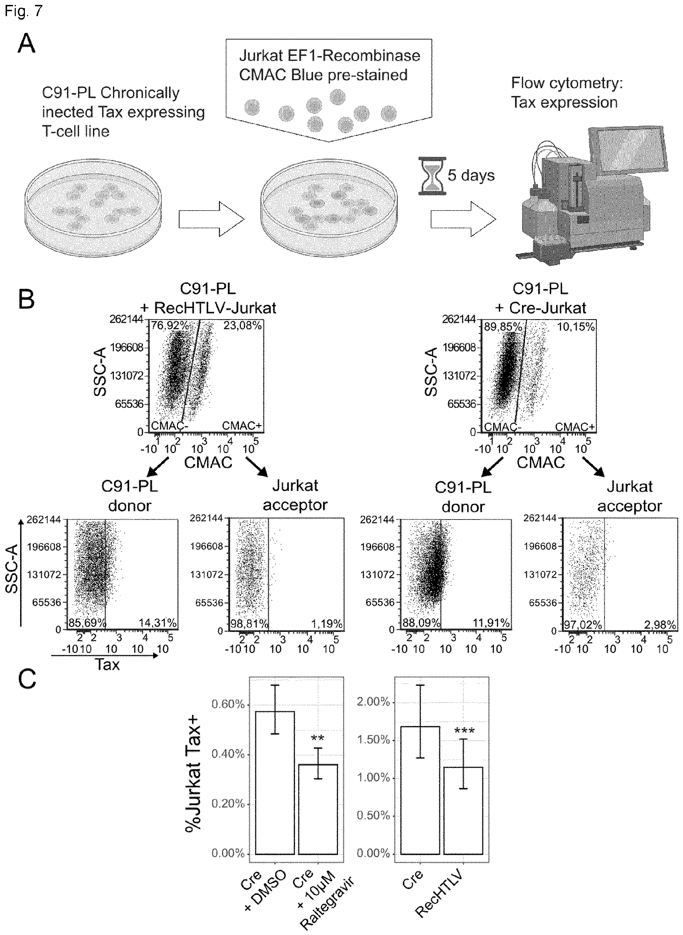

**Fig. 7** A) Schematic representation of the experimental work: Chronically HTLV-1 infected C91-PL donor cells were co-cultured for 5 days with CMAC-Blue pre-stained Jurkat acceptor T-cells which constitutively express the RecHTLV recombinase or Cre as a control. Infected Jurkat T-cells were detected by intracellular FACS staining of Tax protein. B) Representative dot plots obtained by flow cytometry showing the gating strategy for co-culture of Jurkat acceptor cells (CMAC+) and C91-PL donor cells (CMAC-). The two populations are discriminated based on the CMAC staining and Tax expression is further detected in the distinct cell populations. C) Quantification of the frequency of Tax+ cell in Jurkat (CMAC+) acceptor cells. Left: Integrase inhibitor Raltegravir compared to the solvent control DMSO; Right: Jurkat cells expressing either Cre or RecHTLV. For statistical analysis, the fraction of C91-PL Tax+ cells in the co-culture was considered. Error bars show 95% confidence intervals (n=8, ** p<0.01; *** p<0.001, linear mixed model followed by a t-test). Parts of the figure were created with BioRender.com.

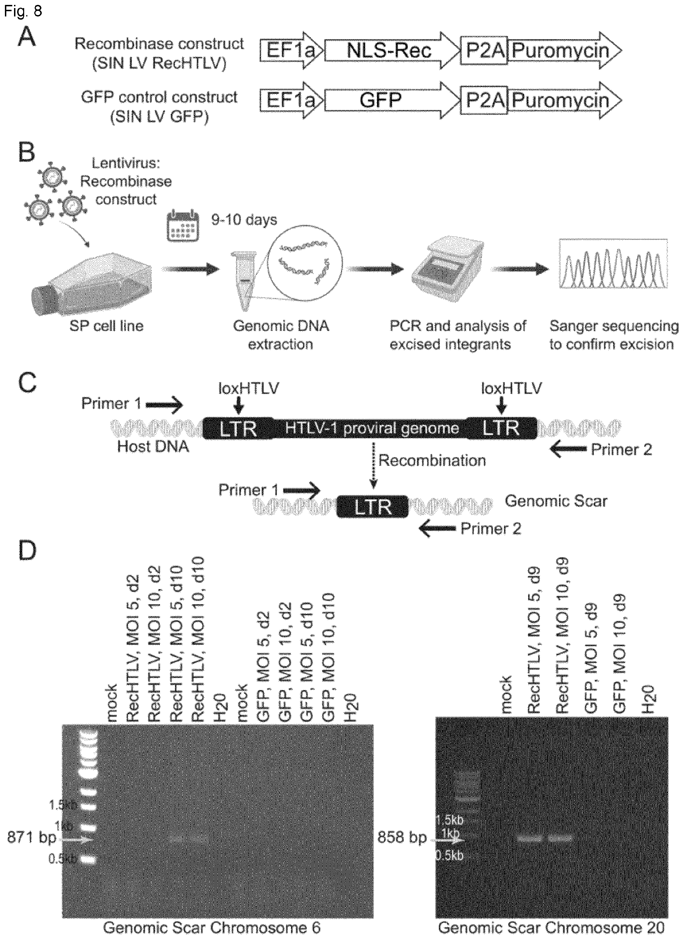

**Fig. 8** A) Schematic representation of the used lentiviral expression constructs SIN LV RecHTLV and SIN LV GFP. The EF1a promoter drives the expression of the RecHTLV, the HTLV-1 specific recombinase fused to nuclear localization signal (NLS-Rec) or GFP, followed by a P2A peptide, which allows the expression of a puromycin resistance gene. B) Experimental workflow: The HTLV-1-infected SP cell line was transduced with lentiviral vectors expressing RecHTLV or GFP. After 9-10 days, genomic DNA was extracted and a PCR was performed followed by Sanger sequencing to confirm the correct excision of the HTLV-1 provirus. C) Scheme of the HTLV-1 provirus integrated into the genome of the SP cells. The black arrows indicate the position of the primers used, which bind in the genomic DNA of the host cell. Upon recombination on the loxHTLV sites by RecHTLV, the proviral genome will be excised leaving a "genomic scar" consisting of one LTR sequence. D) PCR on genomic DNA of recombinase-treated SP cells with primers as indicated in panel B for the proviruses in chromosome 6 and 20. Marked with red arrows are the PCR products which indicate excision of proviral DNA. MOI indicates multiplicity of infection of the recombinase or GFP constructs and d indicates the days post transduction. Parts of the figure were created with BioRender.com.

### Examples

### Results

### Target site selection

The target sites for Cre-like recombinases typically consist of an 8bp spacer sequence flanked by 13bp inverted repeats, which can harbour some positions of asymmetry (Sarkar *et al,* 2007; Karpinski *et al,* 2016; Meinke *et al,* 2016). In order to identify a suitable recombinase target site, we applied the SeLOX algorithm (Surendranath *et al,* 2010) to a collection of 949 LTR sequences deposited in the HTLV-1 Molecular Epidemiology Database (Araujo *et al,* 2012). From this data, we identified the most conserved 34-mers, restricting the results to sequences present in at least 80% of all the deposited LTRs. After discarding sequences containing mononucleotide repeats of 5 identical bases or more, we obtained a set of eight candidate target sites. To avoid possible unwanted recombination on human off-target sites, we searched for all occurrences of sequences resembling the candidate target sites (reference assembly GRCh38 from December 2013). We allowed up to two mismatches per half-site and disregarded mismatches in the 8 bp spacer regions. As a result, we discarded five candidates with potential off-target risk. To select the most suitable of the three remaining target sites, we compared sequences of their half-sites to target sites of previously evolved recombinase libraries (Buchholz & Stewart, 2001; Sarkar *et al,* 2007; Karpinski *et al,* 2016; Lansing *et al,* 2020, 2022). We nominated the target site with the lowest number of mismatches against one of the previous target sites and named it loxHTLV (Fig. 1A). The final target site is found at nucleotide positions from 317 to 350, and from 8,597 to 8,630 (GenBank entry AB513134.1). loxHTLV is highly conserved and found without mismatches in 86% of the sequences from the HTLV-1 Molecular Epidemiology Database. Because loxHTLV is located in the LTR sequences of HTLV-1, flanking the genome of the retrovirus, recombination of these sites would result in the excision of the complete proviral coding sequences from infected cells.

### Directed evolution ofHTLV-1 targeting recombinases

In order to develop a designer-recombinase able to recombine the loxHTLV site, we performed Substrate-linked Directed Evolution (SLiDE) (Materials and Methods) (Buchholz & Stewart, 2001; Karpinski *et al,* 2016; Lansing *et al,* 2020, 2022). SLiDE is performed in *E. coli* and links the excision activity of recombinases to the plasmid that encodes them. In each evolution cycle, the recombinases that successfully excise the target site were selected and amplified by PCR thus introducing variability in the library. Amplified recombinases were then inserted back into the evolution plasmid (pEVO) to allow successive adaptation.

In total, we performed 181 evolution cycles via 7 subsites in order to obtain a library able to efficiently recombine the loxHTLV sequence at low recombinase expression levels, indicating that clones with high activity had been selected during SLiDE (Fig. 1B and C).

### Selection of RecHTLV recombinase clone

In order to enrich for recombinases that do not have adverse effects when expressed in mammalian cells, the final library was inserted into a lentiviral vector that allowed the expression of the recombinases from a tetracycline inducible promoter (Fig. 2A). Following infection of HeLa cells at low multiplicity of infection to ensure that most cells express a single recombinase clone, the library was expressed by the addition of doxycycline to the media for 7 days, ensuring that expression of the recombinase was well tolerated in the cells (Fig. 2A) (Lansing *et al,* 2022). The coding sequences of well tolerated recombinases were then PCR amplified from genomic DNA and inserted into the pEVO vector to evaluate recombination activity in *E. coli.* Recombinase expression was induced by the addition of 10 µg/mL L-Arabinose and the excision activity was tested for 96 clones using a PCR assay. From the 96 clones, 44 showed recombination activity as indicated by the appearance of the recombination-specific band. Sanger sequencing of the clones revealed that some of them shared the same sequence (data not shown). Therefore, we selected the 29 unique active clones for further evaluation in mammalian cell culture. The selected recombinases were inserted into a mammalian expression vector and separately co-transfected into HeLa cells together with a fluorescent reporter plasmid which allows for expression of mCherry if loxHTLV target sites in hoxLTR are recombined to evaluate their activity on the loxHTLV site in mammalian cells (Fig. 2C). The clone G9, hereafter referred to as RecHTLV, showed the highest activity in this assay and was hence selected for further evaluation as candidate HTLV-1 recombinase (Fig. 2C). Other clones A3_A9_C6, A11_E1; B11, B12, C2, D7, D11_F7_F9_G8, E5_E9 (also called E5), F6, F11, F12, G7, G10 and H2 also showed high activity. Clones A11_E1, B11, B12, C2, D7, D1 1_F7_F9_G8, F6, F11, F12 and G9 had the highest activity (more than 60% mCherry expression). F6 and, above all, G9 led to 70% or more mCherry expression.

To confirm that the initial screen for tolerability in HeLa cells was successful, we inserted the RecHTLV coding sequence into the tetracycline inducible lentiviral system described above in which the expression of the recombinases is driven by a tet-inducible promoter and generated HeLa derived cell lines (Fig. 2A). Because these vectors also express GFP, transduced cells can be followed over time. Upon the continuous addition of 100 ng/mL of doxycycline in the medium we observed that the percentage of GFP+ cells dropped only mildly for the cell lines expressing RecHTLV, similar to the GFP-only control (data not shown). These results confirmed the success of the initial screen and that RecHTLV does not affect cellular growth in this system. Therefore, RecHTLV is a well-tolerated candidate HTLV-1 recombinase.

### Mutations acquired during evolution

To get an insight into the mutations that were acquired during evolution, we deep-sequenced the final evolution library using PacBio long-read sequencing technology. We observed that through evolution, the majority of clones in the library had acquired dominating mutations in 35 residues compared to Cre (L5Q, V7L, N10S, P12S, P15L, V23A, K25Q, D29V, M30T, R34H, S38P, K57E, Y77H, A84T, K86N, Q90K, G93A, Q94E, M97T, S108G, K122R, I166V, A175S, K244R, N245Y, A248V, A249V, R259H, L261M, E262Q, E266G, T268A, P307A, N317T, I320S).

As previously described, many of these mutations are residues that have direct contact or proximity to the target site DNA, indicating a putative function of these residues in modulating the selectivity towards the new target site ((Karpinski *et al,* 2016; Sarkar *et al,* 2007; Buchholz & Stewart, 2001). We next sequenced the selected RecHTLV clone G9 using Sanger sequencing and observed that it differs from Cre in 46 residues (*N3K, V7L, N10D, P12S, P15L, V16A, A18V, V23A, S38P, K43E, K57E, L58S, Y77H, A84T, K86N, 188V, Q90K, G93A, Q94E, M97T, S108G, S114T, K122R, Q144R, Q156L, 1166V, A175S, T206A, S226T, V227A, K244R, N245Y, A248V, A249V, P250S, Q255R, R259H, L261M, E262Q, E266G, A267T, T268A, M299L, P307A, N317R, 1320S).* For evolution of E5, 42 mutations had been acquired compared to cre: *N3K, V71, H8Y, N10D, P12S, P15L, V16A, V23A, S38P, K57E, L58S, Y77H, A84T, K86N, 188V, Q90K, G93A, Q94E, M97T, S108G, S110N, K122R, S147A, C155R, 1166V, A175S, K183R, K244R, N245Y, A248V, A249V, Q255R, R259H, L261M, E262Q, E266G, T268A, P307A, N317R, N319D, 1320S and D343Y* (see alignment in Fig. 2F). These are the highest number of changes observed in a Cre-derived recombinase to date, possibly due to the marked differences of loxHTLV when compared to loxP.

The changes indicated in Fig. 2D, namely, V7L, P12S, P15L, V23A, S38P, Y77H, A84T, K86N, Q90K, G93A, Q94E, M97T, S108G, K122R, I166V, A175S, K244R, N245Y, A248V, A249V, R259H, L261M, E262Q, E266G, T268A, N317T and I320S are present in more than 75% of the sequenced clones and, except for N317T, where RecHTLV has N317R, also present in RecHTLV. The changes of P12S, P15L, V23A, S38P, Y77H, A84T, K86N, Q90K, G93A, Q94E, M97T, S108G, K122R, I166V, A175S, K244R, N245Y, A248V, A249V, R259H, L261M, E262Q, E266G, T268A, N317R or N317T, and I320S are present in both E5 and G9/RecHTLV and in more than 75% of the sequenced clones. They are therefore considered to represent the most essential changes.

RecHTLV/G9 and E5 contain most of the mutations that emerged through the library evolution (Fig. 2D and Alignment in Fig. 2F), including mutations previously seen in other Cre-based designer-recombinases (Y77H, S108G, I166V, A175S, I320S), indicating a possible general role of these residues in protein stability (Abi-Ghanem *et al,* 2015; Karpinski *et al,* 2016). We also observed that both E5 and G9/RecHTLV have several changes in the first 20 amino acids (positions 3, 7, 10, 12, 15, 16, 18). The role of the N-terminus of Cre has been debated because wild type Cre can efficiently recombine loxP even when the first 20 amino acids are removed, but it has recently been reported that for evolved Cre-type recombinases these amino acid changes contribute to the stability of the protein (Guillén-Pingarrón *et al,* 2022; Rongrong *et al,* 2005; Warren *et al,* 2008). Hence, the amino acid changes in the first 20 residues might also contribute to protein stability of RecHTLV or E5. In one embodiment, one or more, at most, all of the first 20 N-terminal amino acids of the recombinase of the invention (e.g., according to SEQ ID NO: 12, 13 or 14) are missing.

One of the hotspots of mutations for the recombinases, e.g., RecHTLV is in helix D, particularly at residues on positions 84-94, which have been shown in Cre to be important for recognizing the nucleotide positions close to the loxP spacer. Interestingly, some of these changes were previously seen in the Bred recombinase (Karpinski *et al,* 2016). Nonetheless most of the mutations acquired are unique to RecHTLV possibly conferring specificity to the nucleotide changes in loxHTLV differing from the loxBTR target site. Another mutation hotspot occurs in Helix J, which makes direct contact with the major groove of the DNA, and has been shown to be involved in recognition of the nucleotide positions 8, 9, 10-25, 26 and 27 of the target site (Guo *et al,* 1997; Rüfer & Sauer, 2002; Kim *et al,* 2001). The loxHTLV target sequence is very different at these nucleotide positions when compared to loxP or other lox-like targets and we consistently observe unique changes in this region of the protein, including residues 261 and 267, which were never mutated in any other designer recombinase investigated thus far (Lansing *et al,* 2022, 2020; Sarkar *et al,* 2007; Karpinski *et al,* 2016) (Fig. 2F).

Taken together, these data provide additional insight into the recognition of evolved recombinases to their target site, possibly contributing to efforts for a more rational engineering of Cre-based recombinases towards novel target sites (Schmitt *et al,* 2022; Soni et al., 2020; Abi-Ghanem *et al,* 2013).

### RecHTLV specifically recombines loxHTLV in E. coli

To characterize RecHTLV in more detail, we tested its recombination activity at different expression levels in *E. coli.* The pEVO vector allows for testing the activity of recombinase clones in bacteria at different expression levels by modulating the concentration of L-arabinose added to the growth medium. We expressed RecHTLV using 0, 1, 10, 50, 100 and 200 µg/mL of L-arabinose and loaded the isolated plasmid DNA after digestion with diagnostic restriction enzymes on agarose gels. As expected, we observed an increase of intensity of the band specific for the recombined form of the plasmid, indicating that the loxHTLV target sites were recombined in an L-arabinose dose-dependent fashion (Fig. 2B). We compared the recombination activity of Cre on loxP and recombination of loxBTR by Bred, a recombinase with potent antiretroviral activity in HIV-1 infected cells (Karpinski *et al.,* 2016). (Fig. 2B, target sequences in Fig. 1A). We found that at low levels of expression (0, 1 µg/mL of L-arabinose) Cre recombined loxP proficiently, whereas Bred and RecHTLV had less than 10% recombination activity on their respective target sites at this level of induction. However, when expressed at higher levels, RecHTLV efficiently recombined the loxHTLV sites (> 80% recombination), displaying similar activity as Bred on loxBTR. Overall, RecHTLV shows slightly lower activity compared to Cre on loxP, but similar recombination activity to Brec1 on loxBTR.

### RecHTLV recombines loxHTLV efficiently in HeLa cells

To test the activity of RecHTLV in mammalian cells, we constructed a mammalian reporter vector in which a three SV40 polyA cassette was flanked by two loxHTLV sites preventing the expression of the mCherry gene located downstream. Upon recombination of the loxHTLV sites, the polyA cassette is excised allowing the expression of mCherry driven by the CAG promoter (Fig. 3A). In order to express the recombinases (RecHTLV or Cre as control), we used a plasmid that drives the expression from a cytomegalovirus (CMV) promoter and contained an IRES sequence, allowing linked expression of GFP and therefore enabling to control for transfection efficiencies of the plasmids (Fig. 3A). When both plasmids were co-transfected in HeLa cells, we observed mCherry-positive cells only when RecHTLV was co-transfected with the reporter, whereas no mCherry-positive cells were detected in the Cre transfected cells (Fig. 3B, C).

### RecHTLV recombines loxHTLV in a genomic context

In order to test whether RecHTLV can recombine the loxHTLV sequence in a genomic context, we generated a HeLa reporter cell line where two loxHTLV target sites flank a puromycin cassette followed by an out-of-frame mCherry cassette. Upon recombination, the puromycin cassette is excised, allowing for the expression of mCherry driven by a spleen focus-forming virus (SFFV) promoter (Fig. 4A). To express the recombinases, we transiently transfected a pIRES vector driving expression of the recombinase in addition to GFP, in order to be able to track the transfection efficiency. 48h post transfection we detected over 40% mCherry positive cells transfected with RecHTLV (Fig. 4B), signifying that RecHTLV can recombine the loxHTLV sequences in a genomic context. Importantly, expression of Cre recombinase in this reporter cell line did not lead to appreciable numbers of mCherry positive cells (Fig. 4B). In order to confirm the correct excision of the puromycin cassette, we extracted genomic DNA from the transfected cells. PCR assays with primers that flank the loxHTLV sites produced a band pattern consistent with the correct excision via the loxHTLV sites (Fig. 4C). Furthermore, sequencing of the PCR band confirmed the exact nucleotide sequence expected from recombinase-mediated excision. We conclude that RecHTLV is active in human cells and efficiently excises sequences flanked by two loxHTLV sites from the genome.

### Experimental detection of potential off target sites

In order to investigate the specificity of RecHTLV, we bioinformatically screened the human genome for potential off-target sites based on sequence similarity to loxHTLV. The 3 most closely related sites (Fig. 5A) with 5 and 6 mismatches in the half-site sequences to the loxHTLV sequence, respectively, were tested experimentally in bacteria. We observed no detectable recombination on these three putative off-target sites, even at high recombinase expression levels, indicating that RecHTLV is rather specific and does not recombine human target sites with the closest sequence similarity to loxHTLV (Fig. 5A).

To experimentally identify putative RecHTLV off-targets in human cells with an unbiased approach, we used a chromatin immunoprecipitation followed by sequencing assay (ChIP-seq) (Lansing *et al,* 2022). To this aim, we fused RecHTLV to GFP and stably expressed the fusion-protein in HeLa cells. We used a well-established anti-GFP antibody for the immunoprecipitation of the recombinase to enrich for genomic sequences bound by RecHTLV (Ding *et al,* 2015, 2021; Lansing *et al,* 2022; Poser *et al,* 2008; Hein *et al,* 2015). After pull-down, the DNA fragments were deep sequenced using Illumina NGS sequencing. We identified 112 putative binding sites in the genome from which, based on the piled up reads when comparing the control and test samples, we selected 10 peaks for experimental testing in bacteria. To investigate if the bound sequences can be recombined by RecHTLV, we inserted the 92 bp sequence found around the peak summit twice into the pEVO vector carrying RecHTLV as an excision substrate (Fig. 5B).

After induction of recombinase expression, 9 out of 10 constructs did not show any recombination specific band (Fig. 5C), confirming the high specificity of RecHTLV and validating that sequences bound by a designer-recombinase are not necessarily recombination substrates (Lansing *et al,* 2022). However, one of the tested sites, namely peak 7 (Fig. 5C) displayed a weak signal of the band expected from a recombination product. In order to find the exact 34 bp sequence that is recombined by RecHTLV, we split the 92 bp (SEQ ID NO: 10) into 3 areas, called left, middle and right and inserted these sequences into the pEVO twice as excision substrates. We also tested in the same manner the sequence with the highest similarity to loxHTLV (lox-peak7) (Fig. 5D and E). We observed that only the lox-peak7 sequence showed the recombination specific band, thereby identifying exactly the 34 bp that are recognized by RecHTLV in the genomic context (Fig. 5D). Despite being a potential off-target, the lox-peak7 sequence occurs only one time in the human genome (Nurk *et al,* 2022). Hence, deleterious recombination events involving this sequence are highly unlikely and because the spacer sequence differs from the spacer of loxHTLV, recombination between these two sites is implausible. This result demonstrates that RecHTLV has high specificity and that the ChIP sequencing method is suitable for experimentally identifying putative genomic off-target sites of designer recombinases.

### RecHTLV recombines loxHTLV in the context of the full HTLV-1 LTR sequence

To test whether RecHTLV is able to recombine the loxHTLV sequences in the context of the full-length HTLV-1 LTR, we used the plasmid pHpX, which contains two full size LTR sequences of HTLV-1 flanking the Tax protein coding sequence (Nerenberg *et al,* 1987). For expression of recombinases, we constructed a lentiviral plasmid in which the recombinase coding sequences are under control of the elongation factor-1 alpha (EF1a) promoter. Both plasmids were co-transfected into HEK293T or Jurkat T-cell lines. For both cell lines we observed that the expression of the Tax protein was significantly reduced only when transfected with the plasmid expressing RecHTLV, but not for the empty vector control (Puro, missing recombinase cassette) or Cre transfected cells, indicating RecHTLV-mediated excision of the Tax expression cassette from the plasmid (data not shown). To elucidate if the recombinases are also functional in the context of a full-length HTLV-1 proviral construct, we performed transient assays using a plasmid (X1MT) containing the entire genome of HTLV-1 (Derse *et al,* 1997). Co-transfection together with the recombinase expressing plasmids (Fig. 6A) revealed that expression of Gag (detected with a Gag p19 antibody) was significantly reduced in the presence of RecHTLV compared to the empty vector (Puro) or Cre controls (Fig. 6B). As an additional control, we performed the same assay using the HTLV-1 packaging plasmid pCMV-HT1M (Derse *et al,* 2001). In this case, expression of the viral proteins is driven by a CMV promoter while the 5' LTR has been partially deleted, therefore lacking the loxHTLV site (Fig. 6A). In contrast to the full-length proviral sequence, we observed for the 5' LTR truncated provirus that expression of Gag was not reduced when RecHTLV was co-expressed, implying that RecHTLV-mediated reduction of Gag expression worked through recombination of the loxHTLV target sites (Fig. 6C). We conclude that RecHTLV can recombine loxHTLV in the context of the full-length HTLV-1 genome.

### RecHTLV expression reduces HTLV-1 infection of Jurkat T-cells

Next, we wanted to test if the expression of RecHTLV could reduce HTLV-1 infection in Jurkat T-cells. To this end, we generated Jurkat cell lines that continuously express RecHTLV under control of the EF1a promoter, as well as control lines expressing Cre. Because HTLV-1 infection is dependent on close cell-cell contacts and hardly occurs from free viral particles (Fan *et al,* 1992; Pique & Jones, 2012; Alais *et al,* 2015), we co-cultured the recombinase-expressing Jurkat T-cell lines with the chronically infected C91-PL cell line, which can produce infective viral particles (Alais *et al,* 2017, 2015; Popovic *et al,* 1983). In order to be able to differentiate Jurkat from C91-PL cells, we stained the Jurkat cell lines with CMAC blue dye prior to co-culture (Fig. 7A and B) (Donhauser *et al,* 2018). As a marker of productive infection, we measured intracellular Tax protein expression by flow cytometry five days post co-culture. As a positive control, we applied the compound Raltegravir, which has been shown to inhibit HTLV-1 integrase activity and therefore blocks *de novo* infection of cells (Seegulam & Ratner, 2011). As expected, Raltegravir reduced Tax levels of Jurkat T-cells compared to the DMSO solvent control (Fig. 7C), presumably because the drug prevented HTLV-1 integration into the Jurkat cell genome. Importantly, we observed a similar reduction of Tax expression in RecHTLV expressing Jurkat T-cells, while this reduction was not observed for cells expressing Cre (Fig. 7B, C). These results indicate that expression of RecHTLV reduces stable integration of HTLV-1 in Jurkat T-cells.

### RecHTLV expression excises the integrated HTLV-1 provirus from infected cells

Finally, we tested if RecHTLV can excise the HTLV-1 provirus from a cell line isolated from an ATL patient. We used the chronically infected SP patient-derived cell line that has been shown to harbour four proviruses with known HTLV-1 integration sites, including two proviruses containing full-length LTRs (Meissner *et al,* 2017). We first verified that our SP cells harbour these integrated proviruses, on chromosome 6 and 20, and that both contained the loxHTLV sequences in their respective LTRs (data not shown). We then transduced the SP cell line with a lentivirus that constitutively expresses the RecHTLV recombinase (SIN LV RecHTLV) or GFP as a negative control (SIN LV GFP) (Fig. 8A). At day 9 or 10 after infection, we extracted genomic DNA from the infected cells and performed PCR reactions with primers flanking the integration site (Figure 8B and C). Consistent with the excision of the provirus, we could detect the genomic scar for both loci, as we obtained PCR bands of the expected size only in the cells expressing the RecHTLV recombinase (Figure 8D). Sequencing of the DNA fragments indeed confirmed the exact and precise removal of the provirus from these loci (data not shown). Hence, RecHTLV is capable of excising the HTLV-1 provirus from patient derived cells.

### Discussion

Up until today, HTLV-1 is an incurable infectious disease. However, the proviral integration in the host genome and the stability of viral sequences in infected cells make HTLV-1 a suitable target for gene editing tools. In this study, we developed a recombinase-based approach for removing the HTLV-1 provirus from infected cells. The RecHTLV recombinase (also designated G9) and the related clone E5, one of the alternative clones identified by the described method, are able to target and efficiently excise a sequence present in the HTLV-1 LTRs in bacteria and human cells. Sequencing of genomic DNA isolated from cells treated with RecHTLV revealed that the editing was successful and precise. Remarkably, RecHTLV harbours 46 mutations in its amino acid sequence when compared to Cre, likely due to the disparity between the loxHTLV target sequence and loxP, which demonstrates the flexibility to use designer recombinases to target sequences, even when they only have remote similarity to the original loxP sequence.

To investigate potential side effects of RecHTLV, we employed an *in-silico-*based and an experimental-based off-target analysis. We could not detect activity of RecHTLV on the predicted potential off-target sequences, nominated based on sequence similarity to the loxHTLV target site. Furthermore, using an experimental unbiased approach, we showed that ChIP-seq can be used to find unspecific binding sites in the genome, with a sensitivity that allowed us to detect an off-target site showing less than 10% of recombination activity. Fortunately, this potential off-target sequence, lox-peak7, occurs in the genome only once, which alleviates the threat of undesired editing. Furthermore, recombination events between lox-peak7 and loxHTLV are highly unlikely since identical spacer sequences are required for recombination and the spacer sequences of lox-peak7 and loxHTLV are different (Meinke *et al,* 2016).

Previous attempts at gene editing in HTLV-1 have focused on disrupting the expression of viral proteins, either by using zinc finger nucleases to target the LTR and thus disrupt its promoter function, or directly by using CRISPR/Cas9 on the viral Hbz gene (Tanaka *et al,* 2013; Nakagawa *et al,* 2018). While the obtained results were impressive, the editing capacity of nucleases relies on the repair machinery of the treated cell. Therefore, the resulting sequence is unpredictable and escape mutants are likely to emerge, as has been documented for targeting HIV-1 with nucleases (Wang *et al,* 2016a, 2016b; Yoder & Bundschuh, 2016; De Silva Feelixge *et al,* 2016). Tanaka *et al.* aimed to target both LTRs and consequently remove the provirus of the infected cell, using a similar approach to ours. Nevertheless, they observed that most of the edited cells had indels in the LTRs and not larger deletions (Tanaka *et al,* 2013). Therefore, also here, the appearance of indels after treatment could be detrimental since the edited virus could still be functional and therefore resistant to further treatment. In contrast, designer-recombinases offer predictability at nucleotide precision making them an excellent tool for excising the provirus from infected cells. Taken together, our data show that designer-recombinases are a promising tool to reverse HTLV-1 infections in human cells.

### Materials and Methods

### Plasmids

The evolution plasmids pEVO-loxHTLV and all the pEVO-loxHTLV subsite plasmids, and the pEVO-lox containing the loxHTLV off-target sites were generated using the Cold Fusion Cloning Kit (System Biosciences). The inserts were PCR amplified using the pEVO as a template and primers containing the new lox sites as an overhang. The backbone was prepared by digesting the pEVO vector with BglII restriction enzyme (NEB). The cold fusion reaction was performed following the manufacturer's protocol.

The pCAGGS-lox-pA-lox-mCherry reporter plasmid used for the study of the activity of RecHTLV in HeLa cells was generated from the plasmids described by Lansing *et al.* 2020 and Lansing *et al.* 2022, originally derived from pCAG-loxPSTOPloxP-ZsGreen, a gift from Pawel Pelczar (Addgene plasmid # 51269 ; http://n2t.net/addgene:51269; RRID:Addgene_51269) (Hermann *et al*, 2014). The target sites were introduced by PCR with overhang primers and were inserted in the pCAGGS reporter using SalI and EcoRI restriction digestion cloning.

The expression vector pIRES-NLS-eGFP was generated from the pIRESneo-Cre Vector as described by Lansing *et al.* 2020. The recombinases were inserted in the pIRES expression plasmid by restriction digest using XbaI and BsrGI.

The SVFF-loxHTLV-puro-mCherry reporter Lentivirus plasmid (pLenti-loxHTLV-reporter) was generated from the bicistronic tagBFP/eGFP reporter obtained from D. Sürün (Sürün *et al,* 2018) and inserted in the plentiCRISPR v2 lentiviral backbone, a gift from Feng Zhang, (Addgene plasmid #52961; http://n2t.net/addgene:52961; RRID:Addgene_52961)(Sanjana *et al,* 2014). Shortly, the eGFP was exchanged with a mCherry cassette using AgeI and XhoI (NEB). A puromycin cassette was flanked by the loxHTLV sites by PCR amplification using the primers and and inserted by golden gate cloning with BsmBI.

The pLentiX inducible system was constructed as described by Lansing *et al.* 2022, a DNA fragment for TRE3G-EF1a-Tet-ON^{®} 3G-P2A-eGFP was inserted into the Lentiviral backbone of the plentiSAMv2, a gift from Feng Zhang (Addgene plasmid #75112 ; http://n2t.net/addgene:75112; RRID:Addgene_75112) (Joung *et al,* 2017) using NheI and KpnI restriction enzymes (NEB). Recombinases were inserted in the pLentiX with restriction enzyme digestion and ligation using the BsrGI-HF and XbaI enzymes (NEB). For the ChIP-seq experiment, the pLentiX was further modified in order to fuse the recombinases to eGFP. Shortly, the GFP cassette was exchanged for a Puromycin cassette using HpaI restriction digest. Furthermore, the GFP cassette was inserted downstream from the XbaI restriction site. The recombinases were PCR amplified with a reverse primer which removes the stop codon from the coding sequence of the recombinases and cloned using BsrGI and XbaI.

The pLenti-EFla-P2A-Puro was derived from the pLentiX vector by exchanging the TRE3G promoter with a minimal EF1a promoter using NheI and AscI. Furthermore, the Tet-On-3G coding sequence and the additional EF1a promoter was removed using XbaI AgeI restriction enzymes. The resultant plasmid pLenti-EF1a-P2A-Puro allows the cloning of recombinases using BsrGI and XbaI restriction digest cloning.

The coding sequence of RecHTLV was codon optimized for mammalian expression and synthesized as a fragment by Twist Bioscience (South San Francisco, CA). The codon optimized RecHTLV was then cloned by digestion with BsrGI and XbaI restriction enzymes to the vectors used for viral experiments (pLenti-EF1a-P2A-Puro).

Furthermore, the following plasmids were used for transient transfection experiments: pcDNA3.1 (Life Technologies); the Tax-1 wildtype expression vector pHpX-Tax (Nerenberg *et al,* 1987); the full-length HTLV-1 proviral clone pCS-HTLV-X1MT (Derse *et al,* 1997); the HTLV-1 packaging vector pCMV-HT1M harbouring a deletion in the 5'LTR, expressing all HTLV-1 gene products under the control of a cytomegalovirus (CMV) promoter (Derse *et al,* 2001).

For delivery of RecHTLV in SP cells a HIV-1-derived replication- incompetent lentiviral vector (SIN LV RecHTLV) was constructed that provides high safety levels due to a split packaging system and self-inactivating (SIN) vector design (Dull *et al,* 1998). The gene sequence encoding for RecHTLV and a P2A peptide puromycin resistance gene expression cassette was placed under the control of an EF1a promotor.

For this purpose, the following sequences with homologous overhangs were amplified by PCR: the coding RecHTLV sequence from pLentiX-EF1a-flag-NLS-G9co-P2A-puro (insert 1); P2A puromycin resistance gene cassette from plasmid 1555 (insert 2); lentiviral plasmid backbone from plasmid 1539. For the lentiviral GFP control vector, insert 1 was replaced by GFP. GFP coding sequence was amplified from plasmid 1455. The lentiviral vector backbone was also amplified. The construction was carried out using the NEBuilder^{®} HiFi DNA Assembly Cloning Kit (New England BioLabs Inc.) according to the manufacturer's instructions.

*Generation of recombinase libraries and evolution strategy.*

The initial library was generated by shuffling previously generated Cre-derived libraries. Substrate-linked protein evolution was performed as described previously (Buchholz & Stewart, 2001; Sarkar *et al,* 2007; Karpinski *et al,* 2016; Lansing *et al,* 2020, 2022) by cloning the library of recombinases into the pEVO vector with the corresponding target sites (starting from the subsites to the final target site).

XL1 blue cells were transformed with the pEVO libraries and grown in 100mL LB medium containing chloramphenicol (25 µg/mL) and the expression of the library was induced overnight by the addition of arabinose in the system. After 14-16h, 10 mL of the culture was used for extraction of the plasmids using the Gene JET Plasmid Miniprep Kit. 500 ng of plasmid DNA were digested using NdeI and AvrII (NEB) restriction enzymes and 25 ng of the digested DNA was used as template for PCR to amplify the active recombinases in the library. The PCR was performed using MyTaq polymerase (Bioline), which introduces mutations due to its lack of proof-reading activity. The PCR product was digested with XbaI and BsrGI and the recombinase band (-1kb) was extracted from an agarose gel. The new insert was then cloned to the pEVO backbone starting therefore a new cycle of evolution.

Every fifth cycle of evolution, shuffling was performed. To this end, 25ng of the NdeI and AvrII digested miniprep containing the recombinase library was PCR amplified using Herculase II Fusion Polymerase (Agilent). The PCR product was purified and sonicated using Covaris M220 to obtain fragments of about 200-300 bp (50 W peak incident power, 200 cycles per second, 150 s treatment time and 20% duty factor). The fragments were reassembled by PCR, using the sonicated fragments as a template. This PCR was followed by a second reassembly PCR where the full-length recombinase was amplified using Herculase II Fusion polymerase. The PCR was purified using the ISOLATE II PCR and Gel Kit (Bioline) following the manufacturer's protocol and the elute was digested using XbaI and BsrGI-HF (NEB) and after gel purification the digested product was used as insert for ligation into the pEVO plasmid to continue evolution.

### Test digest for assessing activity of libraries/recombinases

In order to assess the activity of the libraries during evolution, 500 ng of the miniprep DNA plasmid from the induced cultures were digested with XbaI and BsrGI-HF restriction enzymes (NEB) and 250 ng of the digestion was run in a 0.8% agarose gel stained with RedSafe (Intron Biotechnology). The same digestion was performed in order to test individual clones, using 500 ng of plasmid DNA extracted from 5 mL of induced culture.

### Quantification of recombination from agarose gel

Recombination efficiency was quantified by the ratio of the non-recombined and the recombined band from the test digest of the pEVO plasmid. The gel images were obtained using an Infinity VX2-3026 transilluminator and the Infinity Capt software (Vilber) and the band intensities were calculated using the GelAnalyzer 19.1 (www.gelanalyzer.com) software by Istvan Lazar Jr., PhD and Istvan Lazar Sr., PhD, CSc.

### Deep sequencing of the recombinase libraries

In order to prepare the loxHTLV library for deep sequencing, 500 ng of the pEVO plasmid from the last cycle of evolution were digested with the restriction enzymes NdeI and AvrII (NEB) to select the active variants in the library. The digestion was further desalted with a MF-membrane and transformed into XL1-Blue *E*. *coli* competent cells (Agilent). The bacterial culture was grown for 14-16h in 100 mL LB containing 25 µg/mL of Chloramphenicol. The plasmid DNA was extracted using the Gene Jet Plasmid Miniprep Kit (ThermoFisher) following the manufacturer's protocol. From this plasmid DNA, 5 µg were digested with BsrGI-HF and XbaI (NEB) restriction enzymes and the 1041 bp containing the recombinase library was enriched by twice binding of the pEVO backbone to the custom SPRI-beads (Ramawatar & Schwessinger, 2018). The supernatant was cleaned with Ampure XP beads (Beckman Coulter) and the resulting DNA was quantified using a Qubit HS assay Kit on a Qubit 2.0 Fluorometer (ThermoFisher) and sequenced by the CRTD Deep sequencing facility with a Sequel System 6.0 using the Pacbio HiFi method. Circular consensus sequencing data was generated with PacBio's ccs v3.4.1 and filtered to only keep sequences of length 1034-1200 bp with a minimum predicted accuracy of 99.997% (Phred Score of ~25). The data was converted to FASTA using SAMtools 1.11 and the coding sequences of the recombinases translated to amino acid using the protein2dna:bestfit alignment model of exonerate v2.3.0. Furthermore, the sequences were filtered to guarantee that all start with a methionine and are 344 aa long with grep, sed and awk. Further processing and analysis of the sequencing data was performed in R (v4.1.0) using the dplyr, Sequence tools (https://github.com/ltschmitt/SequenceTools) and ggplot2 packages.

### Cell culture

HeLa Kyoto (MPI-CBG, Dresden) cell lines were cultured in Dulbecco's modified Eagle's medium (DMEM, Gibco) with 10% Fetal Bovine Serum and 1% Penicillin/Streptomycin (Pen/Strep) (Thermo Fisher) at 37°C with 5% CO2 in a humidified incubator. HEK-293T cells were kept in DMEM (GIBCO, Life Technologies, Darmstadt, Germany) containing 10% fetal calf serum (FCS; Capricorn Scientific, Ebsdorfergrund, Germany), L-glutamine (0.35 g/1) and Pen/Strep (0.12 g/l each). The CD4⁺ T-cell line Jurkat (Schneider *et al,* 1977) was cultivated in RPMI 1640 (45%; GIBCO, Life Technologies) and Panserin 401 medium (45%; PAN-Biotech, Aidenbach, Germany), supplemented with 10% FCS, L-glutamine and Pen/Strep. The HTLV-1 *in vitro* transformed CD4⁺ T-cell line C91-PL (Ho et al., 1984) was cultured in RPMI 1640, containing 10% FCS, L-glutamine and Pen/Strep. To confer puromycin resistance, C91-PL cells were transduced with pLenti-EFla-P2A-Puro empty vector as described before (Millen *et al,* 2019) and subsequently cultivated in C91-PL medium containing 1 µg/ml puromycin.

HTLV-1 infected cells (SP) (NIH AIDS Reagent Program, #3059) were cultured using RPMI 1640 (LONZA) containing 2.0 mM L-glutamine (PAN Biotech), 100 U/ml penicillin - 100 mg/ml streptomycin (Merck), 10% FBS (PAN Biotech), 250 U/ml human IL-2 (Biomol) at 37°C with 5% CO2 in a humidified incubator.

### Screen in HeLa cells for tolerated recombinases

The final library was cloned in the pLentiX vector and the reporter loxHTLV cells were infected with the lentiviral particles containing the recombinases. Cells were transduced with a low MOI to ensure that only one recombinase integrated in each cell. Expression of the library was induced during 7 days using 50 ng/mL doxycycline. The GFP+ cells were then sorted by flow cytometry and expanded for 5 days and then sorted again for GFP+mCherry+ in order to retrieve the active recombinases from the library. Approximately 3500 cells were sorted in the second sorting round

### PCR to screen for active clones

Genomic DNA from the pooled sorted cells was extracted using the QIAamp DNA Blood Kit following the manufacturer's protocol. Recombinases were then retrieved by PCR using the high-fidelity Herculase II Phusion DNA polymerase (Agilent) (primers 21-22) and the PCR product was cloned into the pEVO and the bacterial transformation was plated on a chloramphenicol plate. The next day, 96 colonies were picked into a deep-well 96 well plate and grown overnight using Chloramphenicol LB containing 10 µg/mL L-arabinose. A colony PCR was performed using as a template 1 µL of the overnight culture and MyTaq polymerase (Bioline) (primers 82-84). The PCR products were loaded in a 2% agarose gel for analysis.

### Cell culture plasmid transfection assays

The plasmid assay in cell culture to test the on-target activity of the recombinases was performed as follows: 15,000 HeLa cells / well were seeded in a 96 well plate. The day after 100 ng of reporter plasmid and 150 ng of the pIRES expression plasmid were transfected using Lipofectamine 2000 (0.3 µL per well). 48 hours after transfection the cells well analysed in a MacsQuant VYB (Miltenyi). After single cell gating, recombination efficiency was calculated by dividing the percentage of double positive cells (mCherry+GFP+) by the frequency of all GFP+ cells.

The test in the genomic reporter cells was performed as follows: 100,000 cells per well were seeded in a 24-well plate and transfected with the 500ng of pIRES expression plasmid using 1 µL of lipofectamine per well. 48 hours post-transfection half of cells were analysed for the expression of mCherry and GFP using a MacsQuant VYF flow cytometer (Miltenyi) and the other half of the cells were used for genomic DNA extraction using the QIAamp DNA Blood Mini Kit (Qiagen) and following the manufacturer's protocol. 100 ng of genomic DNA was used as template for PCR to detect recombination of the reporter (primers 25-26). For other transfections using HEK293T cells, 24 h prior to transfection, 5^{∗}10⁵ 293T cells were seeded in 6 well plates. Transfection was conferred using *GeneJuice*^{®} transfection reagent (Merck Millipore, Darmstadt, Germany) according to the manufacturer's protocol using a total amount of 2 µg DNA at a 1:1 ratio of the plasmids used.

Jurkat T-cells were transfected as described previously (Mann *et al,* 2014), with minor modifications. Briefly, 5^{∗}10⁶ cells were electroporated with the *Gene Pulser X Electroporation System* (BioRad) at 290 V and 1500 µF, using a total amount of 50 µg DNA (30 µg pLenti-EFla-P2A-Puro and 20 µg pHpX-Tax).

### Lentivirus production

The Lentiviral transfer plasmids were co-transfected using standard Polyethylenimine (PEI) transfection with the Lentiviral gag/pol packaging plasmid (psPAX2, Addgene#12260) and the envelope plasmid VSV-G (pMD2.G, Addgene #12259) in a molar ratio of 3:1:1 in HEK293T cells. 48 hours after transfection, the supernatant containing the viral particles was harvested and filtered (0.45 mm pore-size PVDF-membrane filter, Millipore). The supernatants were either used directly to infect the desired target cells or stored at - 80°C for later usage.

VSV-g pseudotyped lentiviral particles for transduction of SP cells were produced by transient cotransfection of HEK 293T cells with the lentiviral plasmid and the respective packaging plasmids (Dull *et al,* 1998) using TransitLT-1 as a transfection reagent according to the manufacturer's protocol (Mirus Bio LLC). At 72 hours post transfection viral supernatants were collected and passed through 0.2 um pore size filters to ensure removal of any viral aggregates. The viral supernatant was concentrated by ultracentrifugation through a 20% sucrose cushion and resuspended in RPMI medium. For the determination of the titer (IU/ml) HEK 293T cells were transduced with various volumes of vector, after 72h the genomic DNA was isolated and the titre was determined by ddPCR using the primers 73, 74, 75 and primers specific for the housekeeping gene Ribonuclease P/MRP Subunit P30 (*RPP30; PrimePCR*^{™} *ddPCR*^{™} *Copy Number Assay: RPP30, Human, Bio-Rad)*. The supernatants were stored at -80°C for later usage.

### Transduction and infection of cell lines

HeLa Kyoto cells or Jurkat cells were transduced with lentiviral particles generated from the corresponding lentiviral vectors. For the constructs containing a puromycin cassette, 72 hours after transduction, cells were selected with 2 µg/mL puromycin for 7 days.

SP and HEK293T cells were transduced with various amounts of vector in the presence of 5 ug/mL protamine sulphate (Merck) and spinoculated at 450 g for 10 min at ambient temperature. After spinoculation cells were cultivated at 37°C and 5% CO2 until further use. SP cells were selected with 0,25 to 1 ug/mL puromycin for 7 days.

### Toxicity assay

HeLa derived cell lines transduced to -46% RecHTLV and -89% Empty control with a transgene expressing GFP and tet-inducible recombinase system were cultured in tet-free media (Capricorn Scientific, FBS-TET-12A). 15000 cells/ well were seeded in a flat bottom 96 well plate and were continuously induced for 3, 6 or 9 days with 100 ng/mL of doxycycline. The percentage of GFP expressing cells was measured by flow cytometry using a MacsQuant VYB.

### ChIP-seq and qPCR validation

The ChIP-seq experiment was performed using RecHTLV, RecHTLV-H289L (G9) and another clone (E5 and E5- H289L). Recombinases were fused with eGFP and cloned in a modified version of the tetracycline inducible pLentiX vector. HeLa cells were infected with the lentivirus and selected with 2 µg/mL of puromycin 72 hours after transduction for 7 days. Cells were grown in 10 cm dishes and the expression of the fused recombinases with GFP was induced for 24 h with 100 ng/mL of doxycycline. Cells were inspected under the microscope for expression of nuclear GFP to confirm that the construct works. Cells were crosslinked with 1% formaldehyde for 10 minutes and chromatin extraction and shearing were performed using the truChIP^{®} Chromatin Shearing Kit (Covaris) following the manufacturer's protocol for high cell number. Chromatin was sheared using a Covaris M220 sonicator. As input sample, for further qPCR validation, 1% of the sheared chromatin was separated and the rest of the sample was used for immunoprecipitation. The recombinase-protein fusion was immunoprecipitated using a goat GFP antibody (MPI-CBG antibody facility) and Protein G Sepharose beads (Protein G Sepharose. 4 Fast Flow, GE Healthcare). Complexes were washed 1x with low salt immune complex wash buffer, 1x high salt wash buffer, 1x LiCl wash buffer, 1x TE buffer and eluted with elution buffer (1% SDS, 0.1 M NaHCOs). Eluted samples and the input samples were treated with RNAse, reverse crosslinked and further purified using the ISOLATE II PCR and Gel Kit (Bioline). The eluted DNA was sent to the CRTD Deep sequencing facility for library preparation and Illumina sequencing. Libraries for Illumina were prepared using NEBNext Ultra^{™} DNA Library Prep Kit for Illumina from 17-75 ng of ChIP DNA with 15 PCR amplification cycles and size selection using AMPure XP beads and paired-end sequencing was performed on an Illumina HiSeq2000. Sequencing reads were aligned to the GRCh38.p12 human genome assembly using STAR aligner (Dobin & Gingeras, 2015) using ChIP-Seq analysis parameters. Peak calling was performed with Genrich (https://github.com/jsh58/Genrich), using the ENCODE blacklist (v2) (Amemiya *et al,* 2019). All manipulations and comparisons of genomic intervals were performed using BEDTools (Quinlan & Hall, 2010) and visualizations of pile-up reads were generated with the UCSC Genome Browser (Kent *et al,* 2002; Raney *et al,* 2014) directly from BAM files sorted by samtools command line tool (Li *et al,* 2009).

### Recombination assay of ChIP-seq selected peaks in bacteria

Ten peaks were selected for further recombination testing in bacteria in a plasmid-based assay. 92 bp around the summit of the peaks were cloned twice in a modified version of the pEVO vector using BglII and PciI restriction sites. The RecHTLV recombinase was cloned in the pEVO using BsrGI and XbaI restriction sites. After transformation in XL1blue E. coli the expression of the recombinase was induced using a concentration of 200 µg/mL L-arabinose. Plasmid extraction was performed and recombination was analysed using test digestion (see above).

### Western Blot

Transfected cells were resuspended at the indicated time points after transfection (48 or 72 h) in lysis buffer [150 mM NaCl, 10 mM Tris/HCl (pH 7.0), 10 mM EDTA, 1% Triton^{™} X-100, 2 mM DTT and protease inhibitors leupeptin, aprotinin (20 µg/ml each) and 1 mM phenylmethylsulfonyl fluoride (PMSF)], subjected to repeated *freeze-and-thaw* cycles between -196°C (liquid nitrogen) and 30°C and additionally sonicated three times for 20 s. Equal amounts of proteins (40 µg) were boiled for 5 min at 95°C in sodium dodecyl sulphate (SDS) loading dye (10 mM Tris/HCl (pH 6.8), 10% glycerine, 2% SDS, 0.1% bromophenol blue, 5% β-mercaptoethanol). SDS-PAGE and immunoblot using *Immobilon*^{®}*-FL* PVDF (Merck Millipore, Billerica, MA, United States) or nitrocellulose transfer membranes (*Whatmann*^{®}, *Protran*^{®}*,* Whatmann GmbH, Dassel, Germany) were performed using standard protocols. Proteins were detected with the following primary antibodies: mouse anti-Tax (derived from the hybridoma cell line 168B17-46-34, provided by B. Langton through the AIDS Research and Reference Reagent Program, Division of AIDS, NIAID, NIH) (Langton *et al,* 1988), mouse monoclonal anti-HTLV-1 gag p19 (TP-7, ZeptoMetrix Corporation), mouse monoclonal anti-FLAG (M2, Sigma), mouse monoclonal anti-Hsp90 α/β (F-8, Santa Cruz Biotechnology), and mouse monoclonal anti-α-Tubulin (T9026, Sigma). Secondary antibodies were anti-mouse Alexa Fluor^{®} 647 (Life Technologies GmbH) or anti-mouse conjugated with horseradish peroxidase (HRP; GE Healthcare, Little Chalfont, United Kingdom). Fluorescence or chemiluminescence signals were detected using the Advanced Fluorescence Imager camera (ChemoStar, Intas Science Imaging GmbH, Göttingen, Germany). Densitometric analysis was performed with the Advanced Image Data Analyzer (AIDA Version 4.22.034, Raytest Isotopenmessgerate GmbH, Straubenhardt, Germany) to compare Tax or Gag expression levels, respectively.

### Co-culture assay between chronically infected C91-PL-Puro donor cells and pre-stained Jurkat-pLenti-EF1a acceptor cells

1^{∗}10⁶ C91-PL-Puro cells were co-cultured with 1^{∗}10⁶ Jurkat-pLenti-EF1a T-cells for 5 days in a 1:1 mixture of C91-PL and Jurkat medium containing puromycin in order to maintain recombinase expression in Jurkat-pLenti-EF1a T-cells. Co-cultures were split twice, at 24 h and 72 h after the onset of the experiment, at a 1:2 ratio. Jurkat-pLenti-EF1a T-cells have been pre-stained with the live cell dye *CellTracker*^{™} *Blue 7-Amino-4-Chlormethylcumarin* (CMAC; Thermo Fisher Scientific) as described earlier (Donhauser *et al,* 2018). As a control, pre-stained Jurkat-pLenti-EF1a T-cells were pre-incubated with 10 µM of the integrase inhibitor Raltegravir for 24 h before initiating the co-culture. Raltegravir was replenished whenever fresh medium was added to the co-culture to keep a final concentration of 10 µM. Analogously, Jurkat-pLenti-EF1a T-cells were treated with DMSO as solvent control. Cells were stained using mouse anti-Tax (Langton *et al,* 1988) and anti-mouse AlexaFluor^{®} 647-conjugated secondary antibodies (Life Technologies GmbH). Briefly, cells were permeabilized with 0.3% Triton^{™} X-100, dispensed in FACS buffer (PBS/1% FCS/2 mM EDTA), for 10 min at room temperature. Staining with primary and secondary antibodies, diluted in FACS buffer, was conferred for 45 min at 37°C with three washing steps in between. Co-cultures were analysed using the BD LSR II flow cytometer (BD Biosciences, San Jose, CA, USA) and cells were discriminated by CMAC-staining (Jurkat-pLenti-EF1a: CMAC-positive; C91-PL-Puro: CMAC-negative) and their different size (FSC/SSC). The percentage of Tax-positive cells within the Tax+ C91-PL donor and the CMAC-positive cells (Jurkat-pLenti-EF1a T-cells) was examined to measure productive infection. For statistical analysis the coefficients were estimated by a linear mixed model followed by a t-test using Satterthwaites's method, provided by the lmerTest package for R (Kuznetsova *et al,* 2017).

### Verification of the HTLV1 integration sites in SP cells

To verify the integration sites on chromosome 6 and 20 and to demonstrate the integrity of the LTR of the proviruses in the SP cells published in (Meissner *et al,* 2017), the sequence regions were amplified verified by sanger sequencing using the same primers.

### Detection of the genomic scar in SP cells

Genomic DNA from cells transduced with RecHTLV or GFP control vector was isolated 9 or 10 days after transduction and the genomic scar was detected by PCR. The PCR fragment was visualized by agarose gel electrophoresis and verified by sanger sequencing using the same primers.

### Reference list

Abi-Ghanem J, Chusainow J, Karimova M, Spiegel C, Hofmann-Sieber H, Hauber J, Buchholz F & Pisabarro MT (2013). Nucleic Acids Res 41: 2394-2403
Abi-Ghanem J, Samsonov SA & Pisabarro MT (2015). J Comput Aided Mol Des 29: 271-282
Alais S, Dutartre H & Mahieux R (2017). Methods Mol Biol 1582: 47-55
Alais S, Mahieux R & Dutartre H (2015). J Virol 89: 10580-10590
Alper H, Fischer C, Nevoigt E & Stephanopoulos G (2005). Proc Natl Acad Sci U S A 102: 12678-12683
Amemiya HM, Kundaje A & Boyle AP (2019). Sci Rep 9: 9354
Araujo THA, Souza-Brito LI, Libin P, Deforche K, Edwards D, de Albuquerque-Junior AE, Vandamme A-M, Galvao-Castro B & Alcantara LCJ (2012). PLoS One 7: e42123
Boxus M & Willems L (2009). Br J Cancer 101: 1497-1501
Buchholz F & Stewart AF (2001). Nat Biotechnol 19: 1047-1052
Coffin JM, Hughes SH & Varmus HE (1997) The Interactions of Retroviruses and their Hosts. In Retroviruses, Coffin JM Hughes SH & Varmus HE (eds) Cold Spring Harbor (NY): Cold Spring Harbor Laboratory Press
De Silva Feelixge HS, Stone D, Pietz HL, Roychoudhury P, Greninger AL, Schiffer JT, Aubert M & Jerome KR (2016). Antiviral Res 126: 90-98
Derossi D, Calvet S, Trembleau A, Brunissen A, Chassaing G & Prochiantz A (1996). J Biol Chem 271: 18188-18193
Derossi D, Joliot AH, Chassaing G & Prochiantz A (1994). J Biol Chem 269: 10444-10450
Derse D, Hill SA, Lloyd PA, Chung Hk null & Morse BA (2001). J Virol 75: 8461-8468
Derse D, Mikovits J & Ruscetti F (1997). Virology 237: 123-128
Ding L, Paszkowski-Rogacz M, Mircetic J, Chakraborty D & Buchholz F (2021). Life Sci Alliance 4: e202000792
Ding L, Paszkowski-Rogacz M, Winzi M, Chakraborty D, Theis M, Singh S, Ciotta G, Poser I, Roguev A, Chu WK, et al (2015). Cell Systems 1: 141-151
Dobin A & Gingeras TR (2015). Curr Protoc Bioinformatics 51: 11.14.1-11.14.19
Donhauser N, Heym S & Thoma-Kress AK (2018). Front Microbiol 9: 400
Donovan PJ & Gearhart J (2001). Nature 414: 92-97
Dull T, Zufferey R, Kelly M, Mandel RJ, Nguyen M, Trono D & Naldini L (1998). J Virol 72: 8463-8471
Edelmann GM, Meech R, Owens GC, Jones FS (2000). PNAS 97, 3038-3043
Elliott G & O'Hare P (1997). Cell 88: 223-233
Eroshenko N & Church GM (2013). Nat Commun 4: 2509
Fan N, Gavalchin J, Paul B, Wells KH, Lane MJ & Poiesz BJ (1992). J Clin Microbiol 30: 905-910
Fawell S, Seery J, Daikh Y, Moore C, Chen LL, Pepinsky B & Barsoum J (1994). Proc Natl Acad Sci U S A 91: 664-668
Furukawa Y, Kubota R, Tara M, Izumo S & Osame M (2001). Blood 97: 987-993
Guillén-Pingarrón C, Guillem-Gloria PM, Soni A, Ruiz-Gómez G, Augsburg M, Buchholz F, Anselmi M & Pisabarro MT (2022). Comput Struct Biotechnol J 20: 989-1001
Guo F, Gopaul DN & van Duyne GD (1997). Nature 389: 40-46
Hartenbach S & Fussenegger M (2006). Biotechnol Bioeng 95: 547-559
Hauber I, Hofmann-Sieber H, Chemnitz J, Dubrau D, Chusainow J, Stucka R, Hartjen P, Schambach A, Ziegler P, Hackmann K, et al (2013). PLoSPathog 9: e1003587
Hein MY, Hubner NC, Poser I, Cox J, Nagaraj N, Toyoda Y, Gak IA, Weisswange I, Mansfeld J, Buchholz F, et al (2015). Cell 163: 712-723
Hermann M, Stillhard P, Wildner H, Seruggia D, Kapp V, Sanchez-Iranzo H, Mercader N, Montoliu L, Zeilhofer HU & Pelczar P (2014). Nucleic Acids Res 42: 3894-3907
Joung J, Konermann S, Gootenberg JS, Abudayyeh OO, Platt RJ, Brigham MD, Sanjana NE & Zhang F (2017). Nat Protoc 12: 828-863
Karpinski J, Hauber I, Chemnitz J, Schäfer C, Paszkowski-Rogacz M, Chakraborty D, Beschorner N, Hofmann-Sieber H, Lange UC, Grundhoff A, et al (2016). Nat Biotechnol 34: 401-409
Kent WJ, Sugnet CW, Furey TS, Roskin KM, Pringle TH, Zahler AM & Haussler and D (2002). Genome Res 12: 996-1006
Kim ST, Kim GW, Lee YS & Park JS (2001). J Cell Biochem 80: 321-327
Kuznetsova A, Brockhoff PB & Christensen RHB (2017). J Stat Soft 82
Langton B, Sliwkowski M, Tran K, Knapp S, Keitelmann E, Smith C, Wallingford S, Liu H, Ralston J & Brandis J (1988). Med Virol 8: 295
Lansing F, Mukhametzyanova L, Rojo-Romanos T, Iwasawa K, Kimura M, Paszkowski-Rogacz M, Karpinski J, Grass T, Sonntag J, Schneider PM, et al (2022). Nat Commun 13: 422
Lansing F, Paszkowski-Rogacz M, Schmitt LT, Schneider PM, Rojo Romanos T, Sonntag J & Buchholz F (2020). Nucleic Acids Res 48: 472-485
Li H, Handsaker B, Wysoker A, Fennell T, Ruan J, Homer N, Marth G, Abecasis G, Durbin R, & 1000 Genome Project Data Processing Subgroup (2009). Bioinformatics 25: 2078-2079
Lin Q, Jo D, Gebre-Amlak KD & Ruley HE (2004). BMC Biotechnol 4: 25
Macara IG (2001) Transport into and out of the nucleus. Microbiol Mol Biol Rev 65: 570-594, table of contents
Mann MC, Strobel S, Fleckenstein B & Kress AK (2014). Virology 464-465: 98-110
Martin F, Tagaya Y & Gallo R (2018). The Lancet 391: 1893-1894
Meinke G, Bohm A, Hauber J, Pisabarro MT & Buchholz F (2016) C. Chem Rev 116: 12785-12820
Meissner ME, Mendonca LM, Zhang W & Mansky LM (2017). J Virol 91: e00369-17
Millen S, Gross C, Donhauser N, Mann MC, Péloponèse Jr. J-M & Thoma-Kress AK (2019). Front Microbiol 10: 2439
Nakagawa M, Shaffer AL, Ceribelli M, Zhang M, Wright G, Huang DW, Xiao W, Powell J, Petrus MN, Yang Y, et al (2018). Cancer Cell 34: 286-297.e10
Nerenberg M, Hinrichs SH, Reynolds RK, Khoury G & Jay G (1987). Science 237: 1324-1329
Nolden L, Edenhofer F, Haupt S, Koch P, Wunderlich FT, Siemen H & Briistle O (2006). Nat Methods 3: 461-467
Nurk S, Koren S, Rhie A, Rautiainen M, Bzikadze AV, Mikheenko A, Vollger MR, Altemose N, Uralsky L, Gershman A, et al (2022). Science 376: 44-53
Oess S & Hildt E (2000). Gene Ther 7: 750-758
Peitz M, Pfannkuche K, Rajewsky K & Edenhofer F (2002). Proc Natl Acad Sci U S A 99: 4489-4494
Pique C & Jones KS (2012). Front Microbio 3
Popovic M, Lange-Wantzin G, Sarin PS, Mann D & Gallo RC (1983). Proc Natl Acad Sci U S A 80: 5402-5406
Poser I, Sarov M, Hutchins JRA, Hériché J-K, Toyoda Y, Pozniakovsky A, Weigl D, Nitzsche A, Hegemann B, Bird AW, et al (2008). Nat Methods 5: 409-415
Quinlan AR & Hall IM (2010). Bioinformatics 26: 841-842
Ramawatar & Schwessinger B (2018) DNA size selection (>3-4kb) and purification of DNA using an improved homemade SPRI beads solution. protocols.io
Raney BJ, Dreszer TR, Barber GP, Clawson H, Fujita PA, Wang T, Nguyen N, Paten B, Zweig AS, Karolchik D, et al (2014). Bioinformatics 30: 1003-1005
Richard JP, Melikov K, Brooks H, Prevot P, Lebleu B & Chernomordik LV (2005). J Biol Chem 280: 15300-15306
Rongrong L, Lixia W & Zhongping L (2005). Acta Biochim Pol 52: 541-544
Rüfer AW & Sauer B (2002). Nucleic Acids Res 30: 2764-2771
Sanjana NE, Shalem O & Zhang F (2014) I. Nat Methods 11: 783-784
Santoro SW & Schultz PG (2002). Proceedings of the National Academy of Sciences 99: 4185-4190
Sarkar I, Hauber I, Hauber J & Buchholz F (2007). Science 316: 1912-1915
Schambach A, Bohne J, Chandra S, Will E, Margison GP, Williams DA, Baum C (2006). Molecular Therapy 13, 391-400
Scherr M & Eder M (2002). Curr Gene Ther 2: 45-55
Schmitt LT, Paszkowski-Rogacz M, Jug F & Buchholz F (2022) Prediction of designer-recombinases for DNA editing with generative deep learning. 2022.04.01.486669 doi:10.1101/2022.04.01.486669 [PREPRINT]
Schneider U, Schwenk HU & Bornkamm G (1977). Int J Cancer 19: 621-626
Seegulam ME & Ratner L (2011). Antimicrob Agents Chemother 55: 2011-2017
Shimshek DR, Kim J, Hübner MR, Spergel DJ, Buchholz F, Casanova E, Stewart AF, Seeburg PH & Sprengel R (2002). Genesis 32: 19-26
Soni A, Augsburg M, Buchholz F & Pisabarro MT (2020). Sci Rep 10: 13985
Surendranath V, Chusainow J, Hauber J, Buchholz F & Habermann BH (2010). Nucleic Acids Res 38: W293-298
Sürün D, Schwäble J, Tomasovic A, Ehling R, Stein S, Kurrle N, von Melchner H & Schnütgen F (2018). Mol Ther Nucleic Acids 10: 1-8
Tanaka A, Takeda S, Kariya R, Matsuda K, Urano E, Okada S & Komano J (2013). Leukemia 27: 1621-1627
Vivès E, Brodin P & Lebleu B (1997). J Biol Chem 272: 16010-16017
Vivès E, Richard J-P, Rispal C & Lebleu B (2003). Curr Protein Pept Sci 4: 125-132
Wang G, Zhao N, Berkhout B & Das AT (2016a). Mol Ther 24: 522-526
Wang Z, Pan Q, Gendron P, Zhu W, Guo F, Cen S, Wainberg MA & Liang C (2016b). Cell Rep 15: 481-489
Warren D, Laxmikanthan G & Landy A (2008). Proceedings of the National Academy of Sciences 105: 18278-18283
Wattel E, Vartanian JP, Pannetier C & Wain-Hobson S (1995). J Virol 69: 2863-2868
Yoder KE & Bundschuh R (2016). Sci Rep 6: 29530
EP 1 1000751.5
WO 2002/44409
WO 2008/083931
WO 2011/147590
WO 2016/034553

## Claims

1. A nucleic acid encoding a tailored recombinase capable of recombining asymmetric target sequences of SEQ ID NO:1 within the long terminal repeat of proviral DNA of a plurality of HTLV-1 strains, wherein the amino acid sequence of the tailored recombinase has at least 70% sequence identity to a sequence according to SEQ ID NO:13, wherein said tailored recombinase comprises, compared to SEQ ID NO:11, at least the amino acid exchanges selected from the group consisting of P15L, S38P, A84T, Q90K, S108G, K122R, I166V, A175S, N245Y, E266G and T268A.

2. The nucleic acid of claim 1, wherein said tailored recombinase has at least 80%, preferably, at least 90% sequence identity to SEQ ID NO: 13.

3. The nucleic acid of any of the preceding claims, wherein the tailored recombinase further comprises SEQ ID NO: 38.

4. The nucleic acid of any of the preceding claims, wherein said tailored recombinase further comprises at least one, preferably, 2 to 101 of the following defined amino acids in the recited positions compared to SEQ ID NO: 11:
- 3K, 3N, or 3D, preferably, 3K;
- 5L, 5Q or 5P, preferably, 5L;
- 7L or 7I, preferably, 7L;
- 8H, 8P or 8Y, preferably, 8H;
- 9Q or 9H, preferably, 9Q;
- 10S or 10D or 10N, preferably, 10S;
- 12S or 12F or 12T, preferably, 12S;
- 14L or 14S, preferably, 14L;
- 16A or 16V, preferably, 16A;
- 17D or 17G or 17N, preferably, 17D;
- 18A or 18V, preferably, 18V;
- 19T or 19A or 19M, preferably, 19T;
- 23A or 23T, preferably, 23A;
- 25K, 25Q or 25R, preferably, 25K;
- 26N or 26S, preferably, 26N;
- 28M or 28T, preferably, 28M;
- 29D or 29V or 29I, preferably, 29D;
- 30M or 30T or 30V, preferably, 30M;
- 31F or 31L, preferably, 31F;
- 34R or 34H or 34C, preferably, 34R
- 35Q or 35H, preferably, 35Q;
- 39E or 39A, preferably, 39E;
- 43K or 43E or 43R; preferably, 43E;
- 45L or 45F, preferably, 45L;
- 51S or 51A or 51T; preferably, 51S;
- 54A or 54T, preferably, 54A;
- 57 E or 57K, preferably, 57E;
- 58S or 58L, preferably, 58S;
- 59N or 59D, preferably, 59N;
- 62K or 62R, preferably, 62K;
- 63W or 63R, preferably, 63W;
- 68P or 68H, preferably, 68P;
- 77H or 77Y or 77D; preferably, 77H;
- 86N or 86S, preferably, 86N;
- 88V or 88I, preferably, 88V;
- 93A or 93T, preferably, 83A;
- 94E or 94D, preferably, 94E;
- 97T or 97M, preferably, 97T;
- 110S or 110N, preferably, 110S;
- 111N or 111S, preferably, 111N;
- 114T or 114S or 114P, preferably, 114T;
- 125V or 125I, preferably, 125V;
- 132K or 132R, preferably, 132K;
- 136A or 136P, preferably, 136A;
- 140T or 140A, preferably, 140T;
- 144R, 144Q or 144L, preferably, 144R;
- 147S, 147T, 147P or 147A, preferably, 147S;
- 149M or 149L, preferably, 149M;
- 151N, 151T, 151D or 151S; preferably, 151N;
- 155C or 155R, preferably, 155C;
- 156L, 156K or 156Q, preferably, 156L;
- 160N or 160D, preferably, 160N;
- 183K or 183R, preferably, 183K;
- 185I or 185V, preferably, 185I;
- 196H or 196R, preferably, 196H;
- 206T or 206A, preferably, 206T;
- 207A or 207T, preferably, 207A;
- 219K, 219R or 219X, preferably, 219K;
- 221V or 2211, preferably, 221V;
- 225I, 225V or 225F, preferably, 225I;
- 226S or 226T, preferably, 226S;
- 227V or 227A, preferably, 227A;
- 229G or 229C, preferably, 229G;
- 230V or 230I, preferably, 230V;
- 231A or 231T, preferably, 231A;
- 232D or 232N, preferably, 232D;
- 235N or 235D, preferably, 235N;
- 241R or 241Q, preferably, 241R;
- 244R or 244K, preferably, 244R;
- 247V or 247I, preferably, 247V;
- 248V or 248A, preferably, 248V;
- 249V or 249A, preferably, 249V;
- 250S or 250P, preferably, 250S;
- 253T or 253A, preferably, 253T;
- 254S, 254K, 254N or 254C, preferably, 254S;
- 255R or 255Q, preferably, 255R;
- 258T or 258A, preferably, 258T;
- 259H, 259Y or 259G, preferably, 259H;
- 261M or 261L, preferably, 261M;
- 262Q or 262R, preferably, 262Q;
- 263G or 263K, preferably, 263G;
- 264I or 264V, preferably, 264I;
- 267A or 267T, preferably, 257A;
- 272I or 272V, preferably, 272I;
- 276K, 276R or 276N, preferably, 276K;
- 277D or 277G, preferably, 277D;
- 278D or 278G, preferably, 278D;
- 280G or 280S, preferably, 280G;
- 281Q, 281R or 281L, preferably, 281Q;
- 299L or 299M, preferably, 299L;
- 304V or 304A, preferably,304V;
- 305S or 305P, preferably, 305S;
- 306I, 306M or 306L, preferably, 306I;
- 307A, 307P or 307V, preferably, 307A;
- 317R or 317T, preferably, 317R;
- 319N or 319D, preferably, 319N;
- 320S, 320T or 320G, preferably, 320S;
- 332T or 332A, preferably, 332T;
- 341D or 341G or 341E, preferably, 341D;
- 342G or 342D, preferably, 342G; and/or
- 343D or 343Y, preferably, 343D.

5. The nucleic acid of claim 4, wherein the tailored recombinase comprises 94E or 94D, preferably, 94E.

6. The nucleic acid of any of claims 4 or 5, wherein the tailored recombinase comprises either
i) 247V and 248V and 244R or
ii) 247I and 248A.

7. The nucleic acid of claim 6, wherein the tailored recombinase comprises 247V and 248V and 244R, wherein, preferably, it also comprises 249V.

8. The nucleic acid of any of the preceding claims, wherein said tailored recombinase comprises at least 34, preferably, all amino acid exchanges compared to SEQ ID NO:11 selected from the group consisting of N3K, V7L, N10D, P12S, P15L, V16A, A18V, V23A, S38P, K43E, K57E, L58S, Y77H, A84T, K86N, I88V, Q90K, G93A, Q94E, M97T, S108G, S114T, K122R, Q144R, Q156L, I166V, A175S, T206A, S226T, V227A, K244R, N245Y, A248V, A249V, P250S, Q255R, R259H, L261M, E262Q, E266G, A267T, T268A, M299L, P307A, N317R, and I320S.

9. The nucleic acid of any of the preceding claims, wherein the tailored recombinase comprises the amino acid sequence according to SEQ ID NO: 13, 14, 26-37 or 39, or a combination of any of said sequences.

10. The nucleic acid of any of the preceding claims, wherein tailored recombinase comprises the amino acid sequence according to SEQ ID NO: 13.

11. A tailored recombinase encoded by the nucleic acid of any of the preceding claims, which is optionally expressed as a fusion protein.

12. A transformed cell comprising the nucleic acid of any of claims 1 to 10, wherein said cell preferably is a stem cell from the hematopoietic lineage.

13. A pharmaceutical composition comprising a nucleic acid of any of claims 1 to 10, a tailored recombinase of claim 11, and/or a transformed cell according to claim 12, wherein the pharmaceutical composition optionally comprises a pharmaceutically acceptable excipient and/or solvent.

14. The pharmaceutical composition of claim 13, wherein the pharmaceutical composition is for use in treatment or prevention of a HTLV-1 infection in a subject, wherein the composition optionally is for use in treatment of adult T-cell leukemia (ATL) and/or HTLV-1-associated myelopathy (HAM).

15. A method of treating a HTLV-1 - infected subject comprising administering an effective amount of the pharmaceutical composition of claim 13 to said subject, wherein the subject optionally has adult T-cell leukemia (ATL) and/or HTLV-1-associated myelopathy (HAM).
